(19) Europäisches Patentamt
European Patent Office
Office européen des brevets

(11) **EP 4 582 104 A1**

(12) **EUROPEAN PATENT APPLICATION**
published in accordance with Art. 153(4) EPC

(43) Date of publication:
**09.07.2025 Bulletin 2025/28**

(21) Application number: **23860317.9**

(22) Date of filing: **29.08.2023**

(51) International Patent Classification (IPC):
***A61K 45/06*** *(2006.01)* ***A61P 35/00*** *(2006.01)*
***A61P 43/00*** *(2006.01)* ***A61K 31/439*** *(2006.01)*
***A61K 31/4439*** *(2006.01)* ***A61K 31/4545*** *(2006.01)*
***A61K 31/4709*** *(2006.01)* ***A61K 31/496*** *(2006.01)*
***A61K 31/506*** *(2006.01)* ***A61K 31/517*** *(2006.01)*
***A61K 31/519*** *(2006.01)* ***A61K 31/5377*** *(2006.01)*

(52) Cooperative Patent Classification (CPC):
**A61K 31/439; A61K 31/4439; A61K 31/4545; A61K 31/4709; A61K 31/496; A61K 31/506; A61K 31/517; A61K 31/519; A61K 31/5377; A61K 45/06; A61P 35/00; A61P 43/00**

(86) International application number:
**PCT/JP2023/031120**

(87) International publication number:
**WO 2024/048555 (07.03.2024 Gazette 2024/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.08.2022 JP 2022136467**

(71) Applicant: **CHUGAI SEIYAKU KABUSHIKI KAISHA**
**Tokyo 115-8543 (JP)**

(72) Inventors:
- **FUJIMURA Takaaki**
  **Kamakura-shi, Kanagawa 247-8530 (JP)**
- **FURUGAKI Ko**
  **Kamakura-shi, Kanagawa 247-8530 (JP)**

(74) Representative: **Vossius & Partner Patentanwälte Rechtsanwälte mbB**
**Siebertstrasse 3**
**81675 München (DE)**

(54) **COMBINATION DRUG**

(57) The present invention relates to a drug, a combination agent, a pharmaceutical composition or a preparation for treating or preventing ALK fusion gene-positive cancer, which comprises an ALK inhibitor and a TNKS inhibitor in combination or comprises a pan-HER inhibitor in combination therewith, and a method for treating or preventing the cancer, as well as an agent for inhibiting the growth of ALK fusion gene-positive cancer which is resistant to the ALK inhibitor on a temporary basis, and the like.

Figure 5

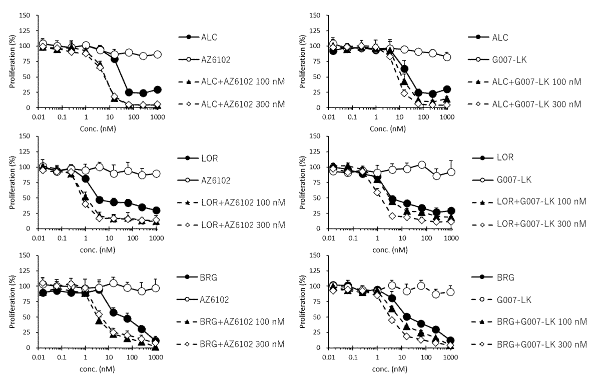


EP 4 582 104 A1

## Description

[Technical Field]

The present invention relates to a drug, a combination agent, a pharmaceutical composition or a preparation which is useful for the treatment or prevention of ALK fusion gene-positive cancer, and comprises an ALK inhibitor and a TNKS inhibitor in combination, as well as a method and a product for treating or preventing target cancer, a drug comprising a triple-drug combination of an ALK inhibitor, a TNKS inhibitor and a pan-HER inhibitor, and the like.

[Background Art]

Anaplastic lymphoma kinase (ALK) is a proto-oncogene of a receptor tyrosine kinase which was identified as a fusion gene in 1994 (Science. 1994 Mar 4; 263(5151): 1281-4.: Non Patent Literature 1).

It has been reported that genetic abnormalities (translocations) in ALK result in the production of abnormal kinases, which are involved in canceration (Non Patent Literature 1). For example, it has been reported that, in lung cancer, ALK is fused to the microtubule associated protein EML4 through chromosomal translocation, and generates EML4-ALK which has active tyrosine kinase activity, thereby gaining oncogenic potential (Cancer Res. 2008 Jul 1; 68(13): 4971-6.: Non Patent Literature 2). It has been reported that compounds having ALK kinase inhibitory activity are useful for the cancers with these genetic abnormalities (Non Patent Literature 2).

Alectinib (generic name: alectinib hydrochloride) is approved in Japan and overseas as an ALK inhibitor.

On the other hand, such inhibitors have been known to have a problem that although immediately after the start of the treatment, a dramatic effect is exhibited against cancer to be treated with the inhibitors, the effects of such inhibitors diminish in some cases as cancer cells acquire resistance through long-term administration (Cell 2010.141(1): 69-8: Non Patent Literature 3).

Tankyrase (TNKS) is an enzyme which is also known as tankyrase 1 and tankyrase 2, and encoded by the TNKS gene and the TNKS 2 gene in humans. TNKS has various roles in cells, such as maintenance of telomere, Wnt signals and mitotic division. It has been reported that genetic abnormalities related to Wnt signals are involved in tumor formation and tumor growth (Oncogene. 2017 Mar; 36(11): 1461-1473.: Non Patent Literature 4). Tankyrase promotes degradation of Axin 1 and Axin 2 which are negative regulators of cancer-promoting β-catenin signaling, so that proliferation of the cells is accelerated (Nature Reviews Drug Discovery volume 11, pages 923-936 (2012): Non Patent Literature 5). TNKS inhibitors have been tested on a clinical/nonclinical basis in colorectal cancer and the like while being used alone or in combination with other anti-cancer agents (Int J Mol Sci. 2021 Jul 8; 22(14): 7330.: Non Patent Literature 6).

An EGFR/ERBB family human epithelial cell proliferation factor receptors (HER) (also known as erythroblastic leukemia viral oncogene homolog (ERBB)), for which the families of EGFR (HER1, ERBB1), HER 2 (ERBB 2), HER 3 (FRBB 3) and HER 4 (ERBB 4) are known, is considered to play an important role in normal regulation of growth, proliferation, differentiation and apoptosis of cell (Sendai Medical Center Journal, Vol. 9, 2019, 23-36: Non Patent Literature 7). A pan-HER inhibitor is a substance having selective inhibitory activity against HER family factor, which exhibits an antitumor effect by inhibiting activation of HER 3 and dimerization of a HER family factor. For example, dacomitinib is used as a therapeutic agent for EGFR gene mutation-positive inoperable or recurrent non-small cell lung cancer.

Suppression of cell proliferation by combined use of different inhibitors against cancer cells that have acquired resistance on a temporary basis through tyrosine kinase inhibitor treatment is examined. For example, an effect of combined use of ceritinib as an ALK inhibitor and a FGFR inhibitor (infigratinib) on a transiently drug-resistant (DTP) strain established by exposing ceritinib to an ALK lung cancer cell strain derived from an ALK lung cancer patient has been reported (Oncogenesis. 2013 Mar; 2(3): e39: Non Patent Literature 8). A synergic effect of combined use of alectinib and a Pan-HER inhibitor (afatinib) for reducing the cell viability of DTP cells prepared by exposing ALK-positive NSCLC to alectinib for a certain period has been reported (npj Precision Oncology volume 6, Article number: 5 (2022), 1-12.: Non Patent Literature 9).

[Citation List]

[Non Patent Literature 1] Science. 1994 Mar 4; 263(5151): 1281-4.
[Non Patent Literature 2] Cancer Res. 2008 Jul 1;68(13):4971-6.
[Non Patent Literature 3] Cell 2010.141(1): 69-8
[Non Patent Literature 4] Oncogene. 2017 Mar; 36(11): 1461-1473
[Non Patent Literature 5] Nature Reviews Drug Discovery volume 11, pages923-936 (2012)

[Non Patent Literature 6] Int J Mol Sci. 2021 Jul 8;22(14):7330.
[Non Patent Literature 7] Sendai Medical Center Journal, Vol. 9, 2019, 23-36
[Non Patent Literature 8] Oncogenesis. 2013 Mar; 2(3): e39
[Non Patent Literature 9] npj Precision Oncology volume 6, Article number: 5 (2022), 1-12.

[Summary of Invention]

In the situations described above, it is desired to provide a therapeutic agent which is effective for treatment of ALK fusion gene-positive cancer, and/or capable of suppressing the proliferation of ALK fusion gene-positive cancer having resistance on a temporary basis.

The present invention provides novel treatment of ALK fusion gene-positive cancer in which a combination drug of an ALK inhibitor and a tankyrase inhibitor (TNKS inhibitor) that has been found from screening with cells which have been acquired from an ALK fusion gene-positive cancer patient and which is resistant to an ALK inhibitor on a temporary basis due to short-term exposure to the inhibitor (drug-tolerant persister cells: DTP cells) suppresses an increase in the amount of β-catenin which is caused by the ALK inhibitor.

That is, the present invention provides a therapeutic agent comprising an ALK inhibitor and a TNKS inhibitor in combination and having an excellent growth suppressive effect on ALK fusion gene-positive cancer.

Another aspect of the present invention provides a therapeutic agent for the cancer comprising a triple-drug combination obtained by further combining a pan-HER inhibitor with the combination of an ALK inhibitor and a TNKS inhibitor.

That is, the present invention relates to the following invention.

<A-1> A drug or pharmaceutical composition for treating or preventing ALK fusion gene-positive cancer, comprising an ALK inhibitor and a TNKS inhibitor in combination.

<A-2> The drug according to <A-1>, further comprising a pan-HER inhibitor in combination.

<A-3> The drug or pharmaceutical composition according to <A-1> or <A-2>, wherein the ALK inhibitor is selected from crizotinib, alectinib, ceritinib, lorlatinib, brigatinib and salts thereof.

<A-4> The drug or pharmaceutical composition according to any one of <A-1> to <A-3>, wherein the ALK inhibitor is selected from alectinib, lorlatinib, brigatinib and salts thereof.

<A-5> The drug or pharmaceutical composition according to any one of <A-1> to <A-4>, wherein the TNKS inhibitor is selected from the group consisting of AZ6102, G007-LK, XAV939, 2X-121, JPI-547, RK-582 and salts thereof.

<A-6> The drug or pharmaceutical composition according to any one of <A-1> to <A-5>, wherein the TNKS inhibitor is AZ6102 or G007-LK.

<A-7> The drug or pharmaceutical composition according to any one of <A-2> to <A-6>, wherein the pan-HER inhibitor is selected from the group consisting of dacomitinib, neratinib, afatinib and salts thereof.

<A-8> The drug or pharmaceutical composition according to any one of <A-2> to <A-7>, wherein the pan-HER inhibitor is dacomitinib or a salt thereof.

<A-9> The drug or pharmaceutical composition according to any one of <A-1> to <A-8>, wherein the ALK fusion gene-positive cancer is selected from the group consisting of non-Hodgkin's lymphoma, neuroblastoma, inflammatory myofibroblastic cytoma, kidney cancer and lung cancer.

<A-10> The drug or pharmaceutical composition according to any one of <A-1> to <A-9>, wherein the ALK fusion gene-positive cancer is non-small cell lung cancer.

<A-11> The drug or pharmaceutical composition according to any one of <A-1> to <A-10>, wherein the ALK fusion gene-positive cancer has not received prior treatment with an ALK inhibitor.

<A-12> The drug or pharmaceutical composition according to any one of <A-1> to <A-11>, wherein the ALK fusion gene-positive cancer has received prior treatment with an ALK inhibitor.

<A-13> The drug or pharmaceutical composition according to any one of <A-1> to <A-12>, wherein the ALK fusion gene-positive cancer is sensitive to ALK.

<A-14> The drug or pharmaceutical composition according to any one of <A-1> to <A-13>, wherein the ALK fusion gene-positive cancer is resistant to an ALK inhibitor on a temporary basis.

<B-1> A drug or pharmaceutical composition for treating or preventing ALK fusion gene-positive cancer, comprising an ALK inhibitor as an active ingredient, which is used in combination with a TNKS inhibitor.

<B-2> The drug or pharmaceutical composition according to <B-1>, wherein the ALK inhibitor is used concurrently with the TNKS inhibitor, or before the start of administration of the TNKS inhibitor.

<B-3> A drug or pharmaceutical composition for treating or preventing ALK fusion gene-positive cancer, comprising a TNKS inhibitor as an active ingredient, which is used in combination with an ALK inhibitor.

<B-4> The drug or pharmaceutical composition according to <B-3>, wherein the TNKS inhibitor is used concurrently with the ALK inhibitor, or after the start of administration of the ALK inhibitor.

<B-5> The drug or pharmaceutical composition according to <B-3> or <B-4>, further comprising a pan-HER inhibitor in combination.

<B-6> The drug or pharmaceutical composition according to any one of <B-1> to <B-5>, wherein the ALK inhibitor is selected from the group consisting of crizotinib, alectinib, ceritinib, lorlatinib, brigatinib and salts thereof.

<B-7> The drug or pharmaceutical composition according to any one of <B-1> to <B-6>, wherein the ALK inhibitor is selected from the group consisting of alectinib, lorlatinib, brigatinib and salts thereof.

<B-8> The drug or pharmaceutical composition according to any one of <B-1> to <B-7>, wherein the TNKS inhibitor is selected from the group consisting of AZ6102, G007-LK, XAV939, 2X-121, JPI-547, RK-582 and salts thereof.

<B-9> The drug or pharmaceutical composition according to any one of <B-1> to <B-8>, wherein the TNKS inhibitor is AZ6102 or G007-LK.

<B-10> The drug or pharmaceutical composition according to any one of <B-5> to <B-9>, wherein the pan-HER inhibitor is selected from the group consisting of dacomitinib, neratinib, afatinib and salts thereof.

<B-11> The drug or pharmaceutical composition according to any one of <B-5> to <B-10>, wherein the pan-HER inhibitor is dacomitinib or a salt thereof.

<B-12> The drug or pharmaceutical composition according to any one of <B-1> to <B-11>, wherein the ALK fusion gene-positive cancer is selected from the group consisting of non-Hodgkin's lymphoma, neuroblastoma, inflammatory myofibroblastic cytoma, kidney cancer and lung cancer.

<B-13> The drug or pharmaceutical composition according to any one of <B-1> to <B-12>, wherein the ALK fusion gene-positive cancer is non-small cell lung cancer.

<B-14> The drug or pharmaceutical composition according to any one of <B-1> to <B-13>, wherein the ALK fusion gene-positive cancer has not received prior treatment with an ALK inhibitor.

<B-15> The drug or pharmaceutical composition according to any one of <B-1> to <B-14>, wherein the ALK fusion gene-positive cancer has received prior treatment with an ALK inhibitor.

<B-16> The drug or pharmaceutical composition according to any one of <B-1> to <B-15>, wherein the ALK fusion gene-positive cancer is sensitive to ALK.

<B-17> The drug or pharmaceutical composition according to any one of <B-1> to <B-16>, wherein the ALK fusion gene-positive cancer is resistant to an ALK inhibitor on a temporary basis.

<C-1> A method for treating or preventing ALK fusion gene-positive cancer, comprising administering to a subject an effective amount of an ALK inhibitor and an effective amount of a TNKS inhibitor in combination.

<C-2> The method according to <C-1>, further comprising administering a pan-HER inhibitor in combination.

<C-3> The method according to <C-1> or <C-2>, wherein the ALK inhibitor is selected from the group consisting of crizotinib, alectinib, ceritinib, lorlatinib, brigatinib and salts thereof.

<C-4> The method according to any one of <C-1> to <C-3>, wherein the ALK inhibitor is selected from the group consisting of alectinib, lorlatinib, brigatinib and salts thereof.

<C-5> The method according to any one of <C-1> to <C-4>, wherein the TNKS inhibitor is selected from the group consisting of AZ6102, G007-LK, XAV939, 2X-121, JPI-547, RK-582 and salts thereof.

<C-6> The method according to any one of <C-1> to <C-5>, wherein the TNKS inhibitor is AZ6102 or G007-LK.

<C-7> The method according to any one of <C-2> to <C-6>, wherein the pan-HER inhibitor is selected from the group consisting of dacomitinib, neratinib, afatinib and salts thereof.

<C-8> The method according to any one of <C-2> to <C-7>, wherein the pan-HER inhibitor is dacomitinib or a salt thereof.

<C-9> The method according to any one of <C-1> to <C-8>, wherein the ALK fusion gene-positive cancer is selected from the group consisting of non-Hodgkin's lymphoma, neuroblastoma, inflammatory myofibroblastic cytoma, kidney cancer and lung cancer.

<C-10> The method according to any one of <C-1> to <C-9>, wherein the ALK fusion gene-positive cancer is non-small cell lung cancer.

<C-11> The method according to any one of <C-1> to <C-10>, wherein the ALK fusion gene-positive cancer has not received prior treatment with an ALK inhibitor.

<C-12> The method according to any one of <C-1> to <C-11>, wherein the ALK fusion gene-positive cancer has received prior treatment with an ALK inhibitor.

<C-13> The method according to any one of <C-1> to <C-12>, wherein the ALK fusion gene-positive cancer is sensitive to ALK.

<C-14> The method according to any one of <C-1> to <C-13>, wherein the ALK fusion gene-positive cancer is resistant to an ALK inhibitor on a temporary basis.

<D-1> An agent for inhibiting the growth of ALK fusion gene-positive cancer resistant to an ALK inhibitor on a temporary basis, comprising an ALK inhibitor and a TNKS inhibitor in combination.

<D-1a> An agent for inhibiting the growth of ALK fusion gene-positive cancer cells resistant to an ALK inhibitor on a temporary basis, comprising an ALK inhibitor and a TNKS inhibitor in combination.

<D-2> The growth inhibiting agent according to <D-1> or <D-1a>, further comprising a pan-HER inhibitor in combination.

<D-3> The growth inhibiting agent according to <D-1> or <D-2>, wherein the ALK inhibitor is selected from the group consisting of crizotinib, alectinib, ceritinib, lorlatinib, brigatinib and salts thereof.

<D-4> The growth inhibiting agent according to any one of <D-1> to <D-3>, wherein the ALK inhibitor is selected from the group consisting of alectinib, lorlatinib, brigatinib and salts thereof.

<D-5> The growth inhibiting agent according to any one of <D-1> to <D-4>, wherein the TNKS inhibitor is selected from the group consisting of AZ6102, G007-LK, XAV939, 2X-121, JPI-547, RK-582 and salts thereof.

<D-6> The growth inhibiting agent according to any one of <D-1> to <D-5>, wherein the TNKS inhibitor is AZ6102 or G007-LK.

<D-7> The growth inhibiting agent according to any one of <D-2> to <D-6>, wherein the pan-HER inhibitor is selected from the group consisting of dacomitinib, neratinib, afatinib and salts thereof.

<D-8> The growth inhibiting agent according to any one of <D-2> to <D-7>, wherein the pan-HER inhibitor is dacomitinib or a salt thereof.

<D-9> The growth inhibiting agent according to any one of <D-1> to <D-8>, wherein the ALK fusion gene-positive cancer is selected from the group consisting of non-Hodgkin's lymphoma, neuroblastoma, inflammatory myofibroblastic cytoma, kidney cancer and lung cancer.

<D-10> The growth inhibiting agent according to any one of <D-1> to <D-9>, wherein the ALK fusion gene-positive cancer is non-small cell lung cancer.

<D-11> The growth inhibiting agent according to any one of <D-1> to <D-10>, wherein the ALK fusion gene-positive cancer has not received prior treatment with an ALK inhibitor.

<D-12> The growth inhibiting agent according to any one of <D-1> to <D-11>, wherein the ALK fusion gene-positive cancer has received prior treatment with an ALK inhibitor.

<D-13> The growth inhibiting agent according to any one of <D-1> to <D-12>, wherein the ALK fusion gene-positive cancer is sensitive to ALK.

<D-14> The growth inhibiting agent according to any one of <D-1> to <D-13>, wherein the ALK fusion gene-positive cancer is resistant to an ALK inhibitor on a temporary basis.

<D2-1> An agent for shrinking ALK fusion gene-positive cancer resistant to an ALK inhibitor on a temporary basis, comprising an ALK inhibitor and a TNKS inhibitor in combination.

<D2-2> The shrinkage agent according to <D2-1>, further comprising a pan-HER inhibitor in combination.

<D2-3> The shrinkage agent according to <D2-1> or <D2-2>, wherein the ALK inhibitor is selected from the group consisting of crizotinib, alectinib, ceritinib, lorlatinib, brigatinib and salts thereof.

<D2-4> The shrinkage agent according to any one of <D2-1> to <D2-3>, wherein the ALK inhibitor is selected from the group consisting of alectinib, lorlatinib, brigatinib and salts thereof.

<D2-5> The shrinkage agent according to any one of <D2-1> to <D2-4>, wherein the TNKS inhibitor is selected from the group consisting of AZ6102, G007-LK, XAV939, 2X-121, JPI-547, RK-582 and salts thereof.

<D2-6> The shrinkage agent according to any one of <D2-1> to <D2-5>, wherein the TNKS inhibitor is AZ6102 or G007-LK.

<D2-7> The shrinkage agent according to any one of <D2-2> to <D2-6>, wherein the pan-HER inhibitor is selected from the group consisting of dacomitinib, neratinib, afatinib and salts thereof.

<D2-8> The shrinkage agent according to any one of <D2-2> to <D2-7>, wherein the pan-HER inhibitor is dacomitinib or a salt thereof.

<D2-9> The shrinkage agent according to any one of <D2-1> to <D2-8>, wherein the ALK fusion gene-positive cancer is selected from the group consisting of non-Hodgkin's lymphoma, neuroblastoma, inflammatory myofibroblastic cytoma, kidney cancer and lung cancer.

<D2-10> The shrinkage agent according to any one of <D2-1> to <D2-9>, wherein the ALK fusion gene-positive cancer is non-small cell lung cancer.

<D2-11> The shrinkage agent according to any one of <D2-1> to <D2-10>, wherein the ALK fusion gene-positive cancer has not received prior treatment with an ALK inhibitor.

<D2-12> The shrinkage agent according to any one of <D2-1> to <D2-11>, wherein the ALK fusion gene-positive cancer has received prior treatment with an ALK inhibitor.

<D2-13> The shrinkage agent according to any one of <D2-1> to <D2-12>, wherein the ALK fusion gene-positive cancer is sensitive to ALK.

<D2-14> The shrinkage agent according to any one of <D2-1> to <D2-13>, wherein the ALK fusion gene-positive cancer is resistant to an ALK inhibitor on a temporary basis.

<F-1> A product comprising: (1) a preparation comprising an ALK inhibitor, (2) a container, and (3) an instruction or label indicating that the ALK inhibitor is to be administered to a subject in combination with at least one TNKS inhibitor for treating ALK fusion gene-positive cancer.

<F-2> The product according to <F-1>, comprising an instruction or label indicating that a pan-HER inhibitor is to be further administered in combination.

<F-3> The product according to <F-1> or <F-2>, wherein the ALK inhibitor is selected from the group consisting of crizotinib, alectinib, ceritinib, lorlatinib, brigatinib and salts thereof.

<F-4> The product according to any one of <F-1> to <F-3>, wherein the ALK inhibitor is selected from the group consisting of alectinib, lorlatinib, brigatinib and salts thereof.

<F-5> The product according to any one of <F-1> to <F-4>, wherein the TNKS inhibitor is selected from the group consisting of AZ6102, G007-LK, XAV939, 2X-121, JPI-547, RK-582 and salts thereof.

<F-6> The product according to any one of <F-1> to <F-5>, wherein the TNKS inhibitor is AZ6102 or G007-LK.

<F-7> The product according to any one of <F-2> to <F-6>, wherein the pan-HER inhibitor is selected from the group consisting of dacomitinib, neratinib, afatinib and salts thereof.

<F-8> The product according to any one of <F-2> to <F-7>, wherein the pan-HER inhibitor is dacomitinib or a salt thereof.

<F-9> The product according to any one of <F-1> to <F-8>, wherein the ALK fusion gene-positive cancer is selected from the group consisting of non-Hodgkin's lymphoma, neuroblastoma, inflammatory myofibroblastic cytoma, kidney cancer and lung cancer.

<F-10> The product according to any one of <F-1> to <F-9>, wherein the ALK fusion gene-positive cancer is non-small cell lung cancer.

<F-11> The product according to any one of <F-1> to <F-10>, wherein the ALK fusion gene-positive cancer has not received prior treatment with an ALK inhibitor.

<F-12> The product according to any one of <F-1> to <F-11>, wherein the ALK fusion gene-positive cancer has received prior treatment with an ALK inhibitor.

<F-13> The product according to any one of <F-1> to <F-12>, wherein the ALK fusion gene-positive cancer is sensitive to ALK.

<F-14> The product according to any one of <F-1> to <F-13>, wherein the ALK fusion gene-positive cancer is resistant to an ALK inhibitor on a temporary basis.

<G-1> A combination of an ALK inhibitor and a TNKS inhibitor or use thereof for producing a drug for treating or preventing ALK fusion gene-positive cancer.

<G-2> The combination or use thereof according to <G-1>, wherein the combination further comprises a pan-HER inhibitor.

<G-3> The combination or use thereof according to <G-1> or <G-2>, wherein the ALK inhibitor is selected from crizotinib, alectinib, ceritinib, lorlatinib, brigatinib and salts thereof.

<G-4> The combination or use thereof according to any one of <G-1> to <G-3>, wherein the ALK inhibitor is selected from alectinib, lorlatinib, brigatinib and salts thereof.

<G-5> The combination or use thereof according to any one of <G-1> to <G-4>, wherein the TNKS inhibitor is selected from the group consisting of AZ6102, G007-LK, XAV939, 2X-121, JPI-547, RK-582 and salts thereof.

<G-6> The combination or use thereof according to any one of <G-1> to <G-5>, wherein the TNKS inhibitor is AZ6102 or G007-LK.

<G-7> The combination or use thereof according to any one of <G-2> to <G-6>, wherein the pan-HER inhibitor is selected from the group consisting of dacomitinib, neratinib, afatinib and salts thereof.

<G-8> The combination or use thereof according to any one of <G-2> to <G-7>, wherein the pan-HER inhibitor is dacomitinib or a salt thereof.

<G-9> The combination or use thereof according to any one of <G-1> to <G-8>, wherein the ALK fusion gene-positive cancer is selected from the group consisting of non-Hodgkin's lymphoma, neuroblastoma, inflammatory myofibroblastic cytoma, kidney cancer and lung cancer.

<G-10> The combination or use thereof according to any one of <G-1> to <G-9>, wherein the ALK fusion gene-positive cancer is non-small cell lung cancer.

<G-11> The combination or use thereof according to any one of <G-1> to <G-10>, wherein the ALK fusion gene-positive cancer has not received prior treatment with an ALK inhibitor.

<G-12> The combination or use thereof according to any one of <G-1> to <G-11>, wherein the ALK fusion gene-positive cancer has received prior treatment with an ALK inhibitor.

<G-13> The combination or use thereof according to any one of <G-1> to <G-12>, wherein the ALK fusion gene-positive cancer is sensitive to ALK.

<G-14> The combination or use thereof according to any one of <G-1> to <G-13>, wherein the ALK fusion gene-positive cancer is resistant to an ALK inhibitor on a temporary basis.

[Brief Description of Drawings]

[Figure 1] Figure 1 shows a graph showing the ratios of cell proliferation of DT cells to non-DT cells (15DT/15cl) under various compounds.

[Figure 2] Figure 2 shows graphs showing the percentages of proliferation of cells with respect to the number of agent-non-treated cells in 15cl and 15DT under alectinib as an ALK inhibitor and AZ6102, G007-LK and XAV939 as TNKS inhibitors in the form of a single agent.

[Figure 3] Figure 3 shows a graph showing the expression levels of proteins after culture in the presence of alectinib in 15cl and 15DT.

[Figure 4] Figure 4 shows a graph showing the expression levels of proteins of cell cytoplasm and nuclei after addition of alectinib in 15cl and 15DT.

[Figure 5] Figure 5 shows graphs showing the percentages of proliferation of cells with respect to the number of agent-non-treated cells in 15cl when alectinib is used in combination with AZ6102 or G007-LK (top), lorlatinib is used in combination with AZ6102 or G007-LK (middle) or brigatinib is used in combination with AZ6102 or G007-LK (bottom).

[Figure 6] Figure 6 shows a graph showing the expression levels of proteins in 15cl when alectinib and AZ6102 are added.

[Figure 7] Figure 7 shows a graph showing the percentages of cell proliferation under a triple-drug combination of alectinib, AZ6102 and dacomitinib in 15cl.

[Figure 8] Figure 8 shows a graph showing a tumor volume (mean + standard deviation) for each administration group when alectinib (2 mg/kg, oral administration once daily for 14 days) is administered in combination with G007-LK (10 mg/kg and 50 mg/kg, oral administration once daily for 14 days) to C. B-17/Icr-scid/scid/Jcl (SCID) mice transplanted with 15cl.

[Figure 9] Figure 9 shows a graph showing a tumor volume (mean + standard deviation) for each administration group when alectinib (2 mg/kg, oral administration once daily for 14 days) is administered in combination with G007-LK (10 mg/kg and 50 mg/kg, oral administration once daily for 14 days) and dacomitinib (10 mg/kg, oral administration once daily for 14 days) to SCID mice transplanted with 15cl.

[Description of Embodiments]

The present invention relates to a drug, a combination agent, a pharmaceutical composition, a preparation and a product for treating or preventing ALK fusion gene-positive cancer, which comprise an ALK inhibitor and a TNKS inhibitor in combination. The present invention also relates to a method for treating or preventing ALK fusion gene-positive cancer, a drug for inhibiting the growth of ALK fusion gene-positive cancer that has acquired resistance on a temporary basis, a drug for reducing the cancer, or a method for inhibiting such growth.

ALK inhibitor

The ALK inhibitor for use in the present invention means a substance capable of directly or indirectly neutralizing, blocking, inhibiting, reducing, or preventing the ALK activity. Examples of the activity of ALK include tyrosine kinase activity. The substance includes a low molecular weight compound, an antibody, an antisense, a transcription inhibitor, a small interfering RNA (siRNA) and the like.

As examples of the low molecular weight compound, first-generation crizotinib (compound name: 3-[(1R-1-(2,6-dichloro-3-fluorophenyl)ethoxy]-5-[1-(piperidin-4-yl)-1H-pyrazol-4-yl]pyridin-2-amine), second-generation alectinib (compound name: 9-ethyl-6,6-dimethyl-8-[4-(morpholin-4-yl)-piperidin-1-yl]-11-oxo-6,11-dihydro-5H-benzo[b]carbazole-3-carbonitrile-hydrochloride), ceritinib (compound name: 5-chloro-N2-(2-isopropoxy-5-methyl-4-piperidin-4-yl-phenyl)-N4-[2-(propane-2-sulfonyl)-phenyl]-pyrimidine-2,4-diamine), and third-generation lorlatinib (compound name: (10R)-7-amino-12-fluoro-2,10,16-trimethyl-15-oxo-10,15,16,17-tetrahydro-2H-4,8-methanepyrazolo[4,3-h][2,5,11]benzoxadiazacyclotetradecine-3-carbonitrile) have been approved up to now, and brigatinib (compound name: {2-[(5-chloro-2-{2-methoxy-4-[4-(4-methylpiperazin-1-yl)piperidin-1-yl]anilinolpyrimidin-4-yl)amino]phenyl}dimethyl-$\lambda^5$-phosphanone) has been approved as the fifth agent in January, 2021.

Alectinib is widely used as a drug of choice. These compounds or salts thereof can be produced according to the methods described in WO2010/143664, WO2004/076412, WO2006/021881, WO2008/073687 and the like. These compounds or salts thereof also include hydrates, various pharmaceutically acceptable solvates, crystalline polymorphs and the like.

Specifically, the "salt" is preferably a pharmaceutically acceptable salt, and examples of thereof include hydrochlorides, hydrobromides, hydroiodides, phosphates, phosphonates, sulfates, sulfonates such as methanesulfonate (mesylates) and p-toluenesulfonate, carboxylates such as acetate, citrate, malate, tartrate, succinate, and salicylate, alkali metal salts such as sodium salts and potassium salts; alkaline earth metal salts such as magnesium salts and calcium salts; and ammonium salts such as ammonium salts, alkylammonium salts, dialkylammonium salts, trialkylammonium salts, and tetraalkylammonium salts.

As a route of administration of the ALK inhibitor for use in the present invention, either oral or parenteral administration is suitably used, but preferably, oral administration is suitably used. The dosage form used for oral administration may be appropriately selected from any dosage form such as a liquid, a powder, granules, a tablet, an enteric coated drug, and a capsule. The ALK inhibitor having such dosage forms is formulated by a method known to those skilled in the art. For example, the compound is formulated by appropriately combining it with a pharmacologically acceptable carrier or medium, specifically, sterile water or saline, vegetable oil, an emulsifier, a suspending agent, a surfactant, a stabilizer, a flavoring agent, an excipient, a vehicle, a preservative, a binder, and the like, and mixing it in the unit dose form required for generally accepted pharmaceutical practices, then by formulating operations such as freeze-drying and tablet compression.

The ALK inhibitor may also be used parenterally in the form of an injection, such as a sterile solution or suspension with water or other pharmaceutically acceptable fluids. The amount of active ingredient in these preparations is selected as appropriate so that the appropriate dose within the indicated range may be administered. A sterile composition for injection may be formulated according to normal preparation practices using a vehicle such as distilled water for injection. Examples of aqueous solutions for injection include saline, an isotonic solution containing glucose or other adjuncts, for example, D-sorbitol, D-mannose, D-mannitol, and sodium chloride, which may be appropriately used in combination with an appropriate solubilizing agent, for example, an alcohol, specifically ethanol, a polyalcohol, for example, propylene glycol and polyethylene glycol, and a nonionic surfactant, for example, polysorbate 80 (TM) and HCO-50. Examples of oily liquids include sesame oil and soybean oil, which may be used in combination with benzyl benzoate and benzyl alcohol as a solubilizing agent. In addition, it may also be suitably blended with a buffering agent, for example, a phosphate buffer solution and a sodium acetate buffer solution, a soothing agent, for example, procaine hydrochloride, a stabilizer, for example, benzyl alcohol and phenol, and an antioxidant.

The dose of the ALK inhibitor according to the present invention may be selected, for example, within the range of 0.0001 mg to 1000 mg per kg of body weight per administration. Alternatively, for example, the dose may be selected within the range of 0.001 mg to 100,000 mg/body per patient. However, the dose of the ALK inhibitor of the present invention is not limited to these doses.

Examples of a more specific dose of the alectinib, the salt thereof, or the hydrate thereof include 20 mg, 40 mg, 60 mg, 80 mg, 120 mg, 160 mg, 220 mg, 240 mg, 300 mg, 460 mg, 600 mg, 760 mg, and 900 mg in free form per dose, twice daily.

The administration period of the ALK inhibitor of the present invention is appropriately determined according to the degree of symptoms and side effects, and can be administered until the cancer is treated or the desired therapeutic effect is achieved. Specifically, the administration may be continuously performed for 7 days to 3 years. Alternatively, there is, for example, a method in which a drug holiday of about 1 to 14 days is set in the administration period, and the administration is performed through 1 to 36 cycles with 2 days to 3 months considered as one cycle. A method is preferable in which the administration is performed through 3 to 24 cycles with 14 to 30 days considered as one cycle.

The Wnt/β-catenin signaling pathway is also called a standard Wnt signaling pathway, which is involved in a variety of physiological processes such as proliferation, differentiation, apoptosis, migration, infiltration and tissue homeostasis. It has been revealed that insufficient regulation of the Wnt/β-catenin cascade contributes to development and progress of some solid tumors and hematological malignant tumors. It is known that impaired control of the Wnt/β-catenin signaling pathway is closely related to development and progress of various types of cancer. Tankyrase (TNKS) promotes degradation of Axin 1 and Axin 2 which are negative regulators of cancer-promoting β-catenin signaling, so that proliferation of the cells is accelerated.

Therefore, it is known that a TNKS inhibitor has a growth suppressive action on cancer cells.

The TNKS inhibitor for use in the present invention means a substance capable of directly neutralizing, blocking, inhibiting, reducing, or preventing the tankyrase activity. The substance includes a low molecular weight compound, an antibody, an antisense, a transcription inhibitor, a small interfering RNA (siRNA, sgRNA) and the like.

Examples of the tankyrase inhibitor include AZ6102, G007-LK, XAV939, 2X-121, JPI-547, RK-582, otaparib, NVP-TNKS656, IWR-1, IWR-2, JW55, WIKI-4, JW-74, LZZ-02 ((E)-3-((4-nitrophenyl)amino)-1-phenylprop-2-ene-1-one), and salts thereof. The structures of these tankyrase inhibitors are as follows.

[Formula 1]

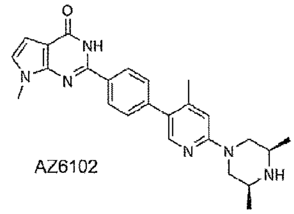

AZ6102

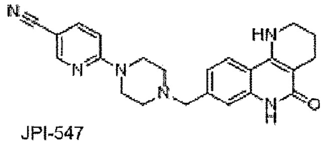

JPI-547

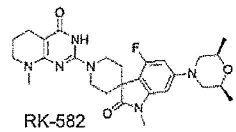

RK-582

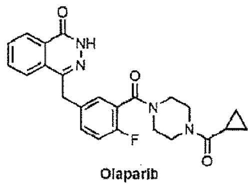

Olaparib

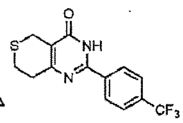

XAV-939

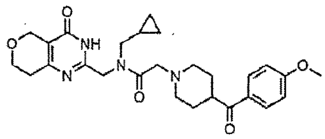

NVP-TNKS656

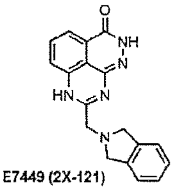

E7449 (2X-121)

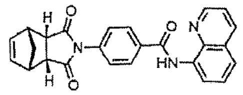

IWR-1

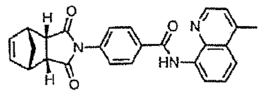

IWR-2

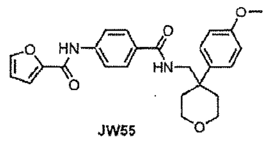

JW55

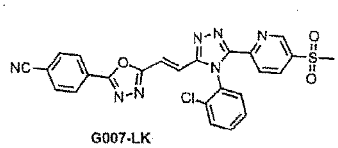

G007-LK

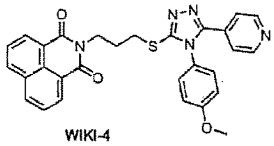

WIKI-4

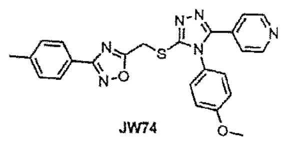

JW74

It is known that the compounds represented by the following general formulae also have TNKS enzyme inhibitory activity (Expert Opin Ther Pat. 2021 Jul; 31(7) 645-661).

[Formula 2]

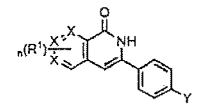
I

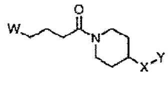
II

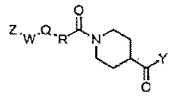
III

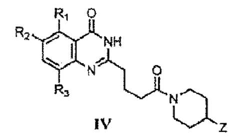
IV

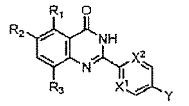
V

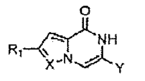
VI

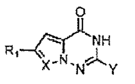
VII

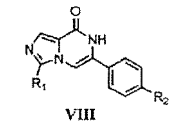
VIII

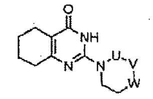
IX

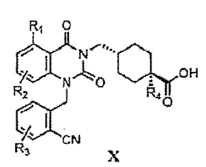
X

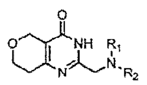
XI

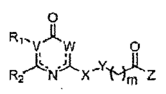
XII

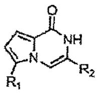
XIII

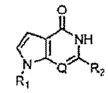
XIV

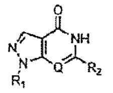
XV

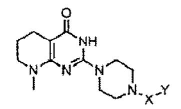
XVI

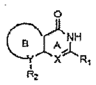
XVII

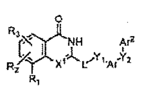
XVIII

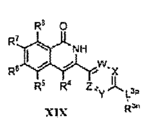
XIX

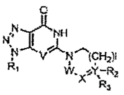
XX

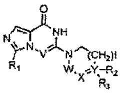
XXI

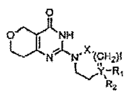
XXII

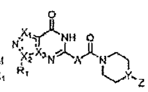
XXIII

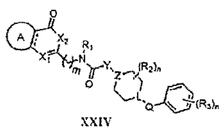
XXIV

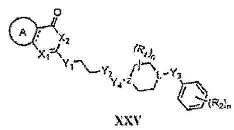
XXV

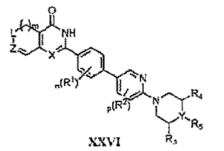
XXVI

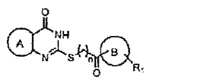
XXVII

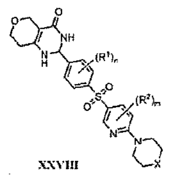
XXVIII

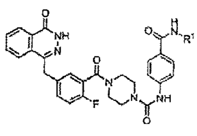
XXIX

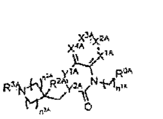
XXX

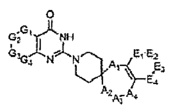
XXXI

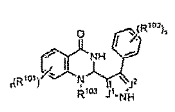
XXXII

[Formula 3]

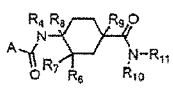
XXXIII

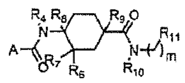
XXXIV

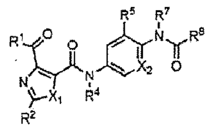
XXXV

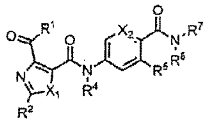
XXXVI

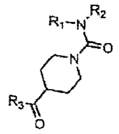
XXXVII

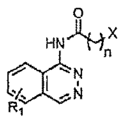
XXXVIII

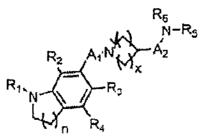
XXXIX

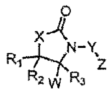
XL

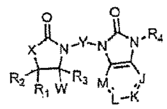
XLI

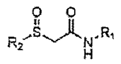
XLII

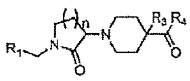
XLIII

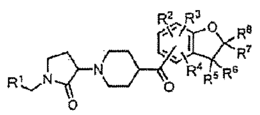
XLIV

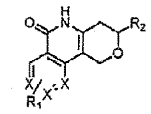
XLV

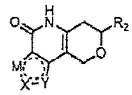
XLVI

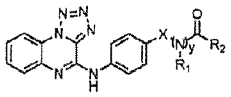
XLVII

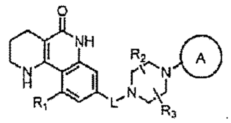
XLVIII

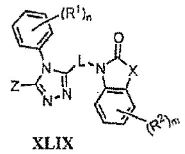
XLIX

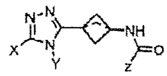
L

Specific examples of compounds covered by the above formulae are as follows.

[Formula 4]

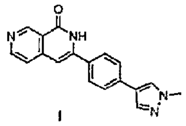

1

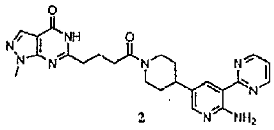

2

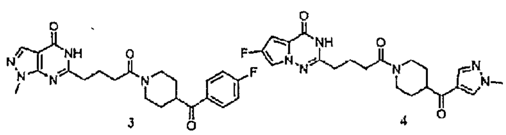

3

4

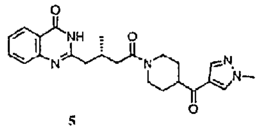

5

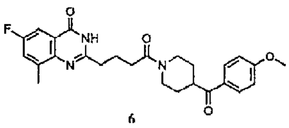

6

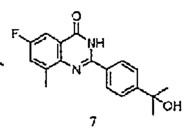

7

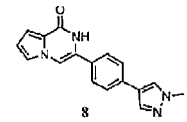

8

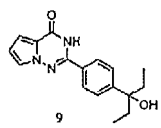

9

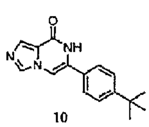

10

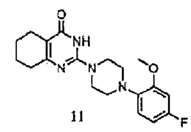

11

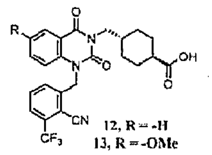

12, R = -H
13, R = -OMe

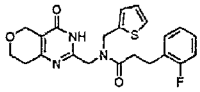

14

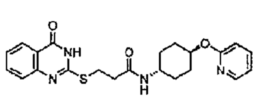

15

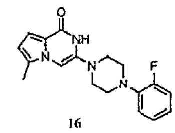

16

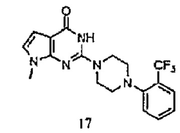

17

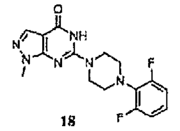

18

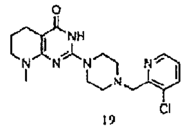

19

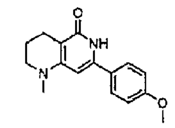

20

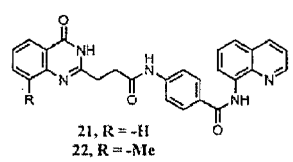

21, R = -H
22, R = -Me

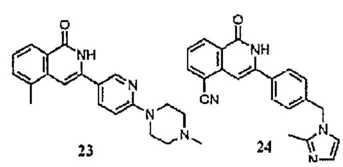

23

24

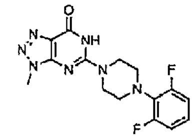

25

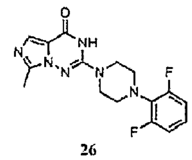

26

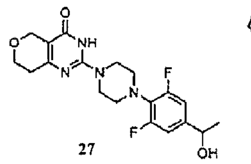

27

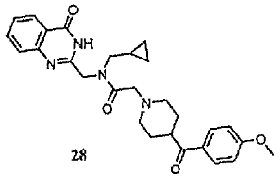

28

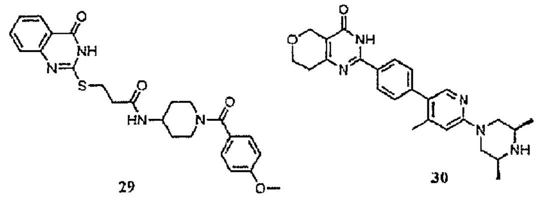

29

30

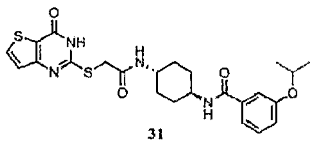

31

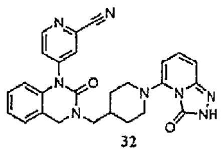

32

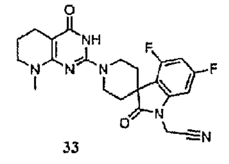

33

[Formula 5]

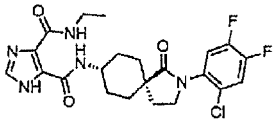
34

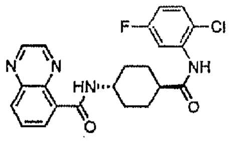
35

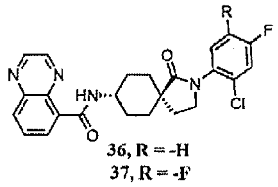
36, R = -H
37, R = -F

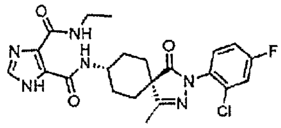
38

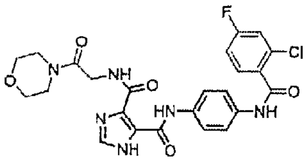
39

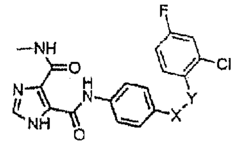
40, X = -NH; Y = -C=O
41, X = -CO; Y = -NH

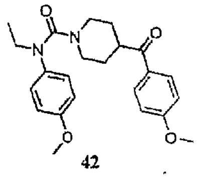
42

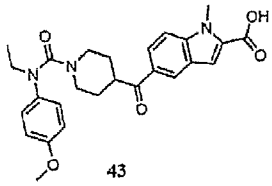
43

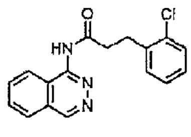
44

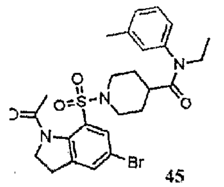
45

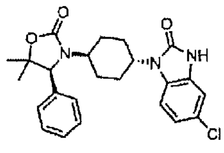
46

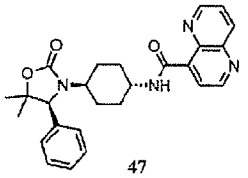
47

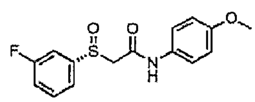
48

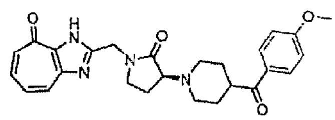
49

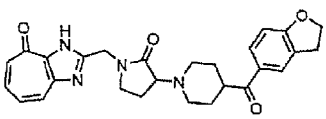
50

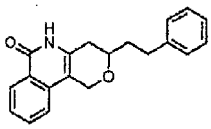
51

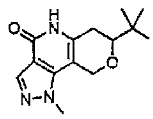
52

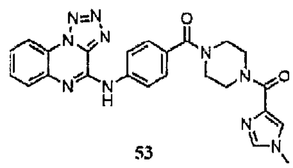
53

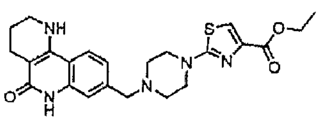
54

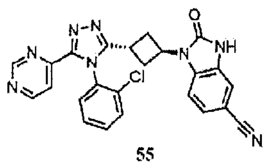
55

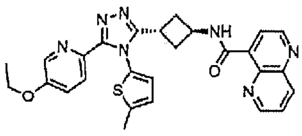
56

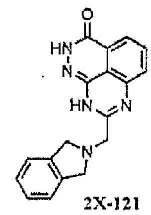
2X-121

AZ6102, G007-LK, XAV939 and salts thereof are preferable.

These compounds or the salts thereof are manufactured and sold as pharmaceutical preparations or can be obtained as reagents for research, and alternatively, they can be produced by a conventional method. These compounds or salts thereof also include salts described in the section of "ALK inhibitor", hydrates, various pharmaceutically acceptable solvates, crystalline polymorphs and the like.

The TNKS inhibitor is formulated according to a conventional method (for example, Remington's Pharmaceutical Science, latest edition, Mack Publishing Company, Easton, U.S.A.) and pharmaceutically acceptable carriers and additives may also be contained. Examples thereof include, but are not limited to, a surfactant, an excipient, a colorant, a flavoring agent, a preservative, a stabilizer, a buffer, a suspending agent, an isotonic agent, a binder, a disintegrant, a lubricant, a flow promoter, and a corrigent, and other commonly used carriers may be used as appropriate. Specifically, suitable examples thereof include trehalose, light anhydrous silicic acid, lactose, crystalline cellulose, mannitol, starch, carmellose calcium, carmellose sodium, hydroxypropyl cellulose, hydroxypropyl methylcellulose, polyvinyl acetal diethylamino acetate, polyvinylpyrrolidone, gelatin, a medium chain triglyceride, polyoxyethylene hydrogenated castor oil 60, refined sugar, carboxymethyl cellulose, corn starch, an inorganic salt, and a polysorbate.

In the present invention, the method for administering the TNKS inhibitor may be carried out by either oral or parenteral administration. Particularly preferred is an administration method by oral administration, and specifically, suitable examples of such administration method include administration by a liquid, a powder, granules, a tablet, an enteric coated drug, a capsule or the like. As an example of administration by injection, the therapeutic drug of the present invention may be administered systemically or locally by intravenous injection, intramuscular injection, intraperitoneal injection, subcutaneous injection, or the like. In addition, the method of administration may be appropriately selected

according to the patient's age and symptoms. For example, in the case of an oral agent, the dose may be selected within the range of 0.1 mg/kg to 100 mg/kg, preferably 0.1 mg/kg to 5 mg/kg per administration. For example, the dose may be selected within the range of 9 mg to 150 mg per patient. However, the dose of the TNKS inhibitor used in the present invention is not limited to these doses.

It has been confirmed in the present invention that an increase in active β-catenin is observed in ALK fusion gene-positive cancer through administration of an ALK inhibitor, and suppressed by a TNKS inhibitor, and a growth suppressive effect on ALK fusion gene-positive cancer cells is obtained by using an ALK inhibitor and a TNKS inhibitor in combination. Therefore, suppression of the activation of β-catenin and an increase in β-catenin may be a major mechanism for the effect.

On the basis of the above-described findings, it is possible to use a substance having an inhibitory action on the Wnt/β-catenin signaling pathway (hereinafter, a β-catenin pathway inhibitor), which contributes to suppression of the activation of β-catenin and an increase in β-catenin, as well as a TNKS inhibitor. That is, as a further aspect of the present invention, a drug, a combination agent, a pharmaceutical composition, a preparation and a product for treating or preventing ALK fusion gene-positive cancer, which comprise an ALK inhibitor and a β-catenin pathway inhibitor in combination, are also included. Examples of the β-catenin pathway inhibitor in the further aspect of the present invention include porcupine (PORCN) inhibitors, Wnt/FZD antagonists, LRP5/6 inhibitors, DVL inhibitor, CK1 agonists, β catenin/TCF inhibitors, and CREB-bound protein (CBP) inhibitors. Specific examples thereof include the following substances (J. Hematol. Oncol. (2020)13: 165, Table 1 or Table 2, Fig. 3).

Examples of the PORCN inhibitor include WNT974, ETC-159, CGX1321, GNF-6231, Wnt-C59, IWP-2, RXC004, and RXC006.

Examples of the Wnt/FZD antagonist include Ipafricept (OMP54F28; IPA), OMP-18R5 (vantictumab), $^{90}\gamma$-OTSA-101, and Fz7-21.

Examples of the LRP5/6 inhibitor include BMD4503-2, and Salinomycin.

Examples of the DVL inhibitor include NSC668036, FJ9, and 3289-8625.

Examples of the CK1 agonist include pyrvinium, and SSTC3.

Examples of the β catenin/TCF inhibitor include PFK115-584, CGP049090, KYA1797K/KY1220, ZINC02092166, NLS-StAx-h, CWP232291 (CWP291), iCRT3/5, iCRT14, and LF-3.

Examples of the CBP inhibitor include PRI-724, ICG001, isoquercittin, GNE-781, 1-(1H-indol-1-yl)ethanone, JW67, JW74, NLS-StAx-h, and ep31670.

pan-HER inhibitor

The pan-HER inhibitor means a substance which irreversibly acts on an EGFR/ERBB family to inhibit activation and signaling of each tyrosine kinase.

The EGFR/ERBB family comprises four types of receptor tyrosine kinases (RTKs): EGFR/HER1/ErbB-1, HER2/ErbB-2/NEU, HER3/ErbB-3 and HER4/ErbB-4. The ligand is different for each receptor, and when the ligand is bound, the EGFR/ERBB family forms a homo or hetero dimer, and activates a kinase domain, and the activation of the kinase domain phosphorylates the binding site of a Src homology 2 (SH2) domain, so that the intracellular signaling pathway is activated.

The pan-HER inhibitor includes a low molecular weight compound, an antibody, an antisense, a transcription inhibitor, a small interfering RNA (siRNA) and the like, which binds to the EGFR/ERBB family receptor to inhibit the receptor, or suppress the activation of the receptor. Examples of the low molecular weight compound include dacomitinib, neratinib, and afatinib.

Dacomitinib or a salt thereof is preferable.

These compounds or the salts thereof are manufactured and sold as pharmaceutical preparations or can be obtained as reagents for research, and alternatively, they can be produced by a conventional method. These compounds or salts thereof also include salts described in the section of "ALK inhibitor", hydrates, various pharmaceutically acceptable solvates, crystalline polymorphs and the like.

The pan-HER inhibitor is formulated according to a conventional method (for example, Remington's Pharmaceutical Science, latest edition, Mack Publishing Company, Easton, U.S.A.) and pharmaceutically acceptable carriers and additives may also be contained. Examples thereof include, but are not limited to, a surfactant, an excipient, a colorant, a flavoring agent, a preservative, a stabilizer, a buffer, a suspending agent, an isotonic agent, a binder, a disintegrant, a lubricant, a flow promoter, and a corrigent, and other commonly used carriers may be used as appropriate. Specifically, suitable examples thereof include trehalose, light anhydrous silicic acid, lactose, crystalline cellulose, mannitol, starch, carmellose calcium, carmellose sodium, hydroxypropyl cellulose, hydroxypropyl methylcellulose, polyvinyl acetal diethylamino acetate, polyvinylpyrrolidone, gelatin, a medium chain triglyceride, polyoxyethylene hydrogenated castor oil 60, refined sugar, carboxymethyl cellulose, corn starch, an inorganic salt, and a polysorbate.

In the present invention, the method for administering the pan-HER inhibitor may be carried out by either oral or parenteral administration. Particularly preferred is an administration method by oral administration, and specifically,

suitable examples of such administration method include administration by a liquid, a powder, granules, a tablet, an enteric coated drug, a capsule or the like. As an example of administration by injection, the therapeutic drug of the present invention may be administered systemically or locally by intravenous injection, intramuscular injection, intraperitoneal injection, subcutaneous injection, or the like. In addition, the method of administration may be appropriately selected according to the patient's age and symptoms. For example, in the case of an oral agent, the dose may be selected within the range of 0.1 mg/kg to 100 mg/kg, preferably 0.1 mg/kg to 5 mg/kg per administration. For example, the dose may be selected within the range of 9 mg to 150 mg per patient. However, the dose of the TNKS inhibitor used in the present invention is not limited to these doses.

Depending on the type and severity of the disease, for example, the preferred daily dose of dacomitinib is within the range of 15 to 45 mg, but is not limited thereto, and may be gradually reduced depending on the degree of symptoms and side effects. In case side effects or the like are observed, the dose is reduced to 30 mg as a primary dose reduction and to 15 mg as a secondary dose reduction. In one example, osimertinib is administered at a dose of 80 mg once daily for 3 consecutive weeks, and in case side effects or the like are observed, the dose is reduced to 4 mg as a primary dose reduction and to 75 mg as a secondary dose reduction. However, other dosage regimens may also be useful.

The TNKS inhibitor can be administered until the target cancer is treated or the desired therapeutic effect is achieved, with the above 3-week administration and 1-week drug holiday considered as one cycle. Alternatively, for the frequency of administration, an administration once a day for a predetermined period of time (for example, 2 weeks) may be usually considered as one cycle, followed by a predetermined drug holiday (for example, 1 week), and then the cycle may be repeated. The number of cycles varies depending on the type and severity of the disease. The treatment is maintained until target cancer is treated or the desired therapeutic effect is achieved by measuring according to a method known in the art.

Drug, Combination, Pharmaceutical Composition, and Method of Treatment and Prevention

An aspect of the present invention is a drug for treating or preventing ALK fusion gene-positive cancer (hereinafter, referred to as target cancer), which comprise an ALK inhibitor and a TNKS inhibitor in combination.

In the above aspect, the "drug for treating or preventing target cancer, which comprises an ALK inhibitor and a TNKS inhibitor in combination" means a drug comprising an ALK inhibitor and a TNKS inhibitor in combination for simultaneous, separate, or sequential administration in the treatment or prevention of target cancer. The drug of the present invention can also be provided in the form of a compounded drug comprising both an ALK inhibitor and a TNKS inhibitor. In addition, the preparation comprising an ALK inhibitor and the preparation comprising a TNKS inhibitor may be provided separately, and these preparations may be used simultaneously or sequentially.

In the present application, the term "in combination" specifically includes the statement of use in combination with other drugs in the dosage and administration of a pharmaceutical preparation. Specifically, the statement of use in combination with a TNKS inhibitor in a package insert for a pharmaceutical preparation of an ALK inhibitor, or the statement of use in combination with an ALK inhibitor in a package insert for a pharmaceutical preparation of a TNKS inhibitor is included.

In the present invention, simultaneous administration refers to the use of an ALK inhibitor and a TNKS inhibitor by administering them at the same time, and it may be administered as a compounded drug, administered as a mixture prepared at the time of administration, or preparations of another form may be administered at the same time. When used by simultaneous administration, it may be administered by different routes or it may be administered by the same route, and the dosage forms for administration may be the same or different.

In the present invention, when an ALK inhibitor and a TNKS inhibitor are administered separately, the order of administration of the ALK inhibitor and the TNKS inhibitor may be: the ALK inhibitor is administered after the administration of the TNKS inhibitor; the ALK inhibitor and the TNKS inhibitor are administered simultaneously; or the TNKS inhibitor is administered after the administration of the ALK inhibitor.

Preferably, the ALK inhibitor and the TNKS inhibitor are administered simultaneously.

Specifically, use in combination with a TNKS inhibitor is stated in a package insert for a pharmaceutical preparation of an ALK inhibitor, and the administration is performed in accordance with the dosage and administration of the ALK inhibitor as specified in the case of use in combination.

In another case, use in combination with an ALK inhibitor is stated in a package insert for a pharmaceutical preparation of a TNKS inhibitor, and the administration is performed in accordance with the dosage and administration of the ALK inhibitor as specified in the case of use in combination.

In the above drug, when the ALK inhibitor and the TNKS inhibitor are contained and provided in separate preparations, the dosage forms of these preparations may be the same or different. For example, both may have a different dosage form than the other, selected from an oral agent, a parenteral agent, an injection, a drip infusion, and an intravenous drip infusion, or both may have the same dosage form selected from an oral agent, a parenteral agent, an injection, a drip infusion, and an intravenous drip infusion. Preferably, the dosage form of both is an oral agent. In addition,

the above drug may further be combined with one or more different preparations.

Note that, in the present invention, the pharmaceutical composition comprises the ALK inhibitor and/or the TNKS inhibitor used in the treatment and/or prevention of the present invention, and may further comprise a pharmaceutically acceptable carrier.

In the present invention, the term "pharmaceutically acceptable carrier" means one or more compatible solid or liquid diluent excipients or encapsulating materials, as previously exemplified, that are suitable for administration to mammals.

In the present invention, a "combination agent" means a form in which an ALK inhibitor and a compound that is a TNKS inhibitor can be used separately for the same subject, or a combination of these compounds where each is formulated without mixing them. For example, packaged doses of the drug are included so that a pharmaceutical preparation of an ALK inhibitor and a pharmaceutical preparation of a TNKS inhibitor can be taken simultaneously.

In the present invention, the "pharmaceutical preparation" comprising an ALK inhibitor and a TNKS inhibitor in combination includes solid preparations such as tablets, capsules, granules, powders, and pills; liquids such as aqueous and non-aqueous oral solutions and suspensions, and parenteral solutions filled in a container adapted for subdividing into individual doses; lyophilized preparations which can be used by dissolving at the time of use; or preparations combining any of these dosage forms, in which these active ingredients are formulated separately, or comprised in the same preparation. The pharmaceutical preparation comprises, per unit dosage form, 150 mg to 800 mg, preferably 150 mg to 400 mg, and particularly preferably 150 mg to 300 mg of alectinib or a salt thereof in free form. Separate preparations of alectinib or a salt thereof include, specifically, a 150 mg capsule preparation, 150 mg, 300 mg, 600 mg tablets and the like.

In another viewpoint, the present invention provides a drug for treating or preventing target cancer, the drug comprising an ALK inhibitor as an active ingredient, which is used in combination with a TNKS inhibitor. The "drug for treating or preventing target cancer, the drug comprising an ALK inhibitor as an active ingredient, which is used in combination with a TNKS inhibitor" means a drug for use in treating or preventing target cancer, the drug comprising an ALK inhibitor as an active ingredient, provided that it is used in combination with a TNKS inhibitor. When the drug comprising an ALK inhibitor as an active ingredient in the present invention is used in combination with a TNKS inhibitor, the drug may be administered simultaneously with the TNKS inhibitor, or may be administered before or after the administration of the TNKS inhibitor. If an ALK inhibitor is administered before or after the administration of a TNKS inhibitor, the timing of the administration may be optimized by measuring the residual concentration of the TNKS inhibitor in the subject. The concentration can be determined based on the results of analyzing a sample collected from the subject by an immunoassay such as ELISA, which is publicly known to those skilled in the art.

In another viewpoint, the present invention provides a drug for treating or preventing target cancer, the drug comprising a TNKS inhibitor as an active ingredient, which is used in combination with an ALK inhibitor. "A drug for treating or preventing target cancer, the drug comprising a TNKS inhibitor as an active ingredient, which is used in combination with an ALK inhibitor" in the present invention means a drug for use in treating or preventing target cancer, the drug comprising a TNKS inhibitor as an active ingredient, provided that it is used in combination with an ALK inhibitor. When the drug comprising a TNKS inhibitor as an active ingredient is used in combination with an ALK inhibitor, the drug may be administered simultaneously with the ALK inhibitor, or may be administered before or after the administration of the ALK inhibitor. If a TNKS inhibitor is administered before or after the administration of an ALK inhibitor, the timing of the administration may be optimized by measuring the residual concentration of the ALK inhibitor in the subject. The concentration can be determined based on the results of separating a sample collected from the subject using a separation device such as various types of chromatography, then analyzing using a method of analysis publicly known to those skilled in the art.

The above invention means that an ALK inhibitor and a TNKS inhibitor are administered or used (hereinafter, simply referred to as "administered") together, and the order of administration, interval of administration, and the like shall not be construed as limited. The invention may also be used as a product in which an ALK inhibitor is combined with a TNKS inhibitor. Furthermore, when an ALK inhibitor is used in combination with a TNKS inhibitor in the present invention, each may be administered at a dose less than that at which either one is used alone, if desired.

The combination of an ALK inhibitor and a TNKS inhibitor which is used for a drug, a combination, a pharmaceutical composition and a treatment/prevention method in the present invention is specifically a combination of an ALK inhibitor that is alectinib or a salt thereof and a TNKS inhibitor selected from AZ6102, G007-LK, XAV939, 2X-121, JPI-547, RK-582 and salts thereof, preferably a combination of an ALK inhibitor that is alectinib or a salt thereof and a TNKS inhibitor selected from AZ6102, G007-LK and salts thereof. In another aspect, it is a combination of an ALK inhibitor selected from alectinib, lorlatinib and salts thereof and a TNKS inhibitor selected from AZ6102, G007-LK, XAV939, 2X-121, JPI-547, RK-582 and salts thereof, preferably a combination of an ALK inhibitor selected from alectinib, lorlatinib and salts thereof and a TNKS inhibitor selected from AZ6102, G007-LK and salts thereof. In still another aspect, it is a combination of an ALK inhibitor selected from alectinib, brigatinib and salts thereof and a TNKS inhibitor selected from AZ6102, G007-LK, XAV939, 2X-121, JPI-547, RK-582 and salts thereof, preferably a combination of an ALK inhibitor selected from alectinib, brigatinib and salts thereof and a TNKS inhibitor selected from AZ6102, G007-LK and salts thereof.

In still another aspect, it is a combination of an ALK inhibitor selected from alectinib, lorlatinib, brigatinib and salts thereof and a TNKS inhibitor selected from AZ6102, G007-LK and salts thereof.

In addition, the combination of an ALK inhibitor, a TNKS inhibitor and a pan-HER inhibitor which is used for a drug, a combination, a pharmaceutical composition and a treatment/prevention method in the present invention is a combination of an ALK inhibitor that is alectinib or a salt thereof, a TNKS inhibitor selected from AZ6102, G007-LK, XAV939, 2X-121, JPI-547, RK-582 and salts thereof and a pan-HER inhibitor selected from the group consisting of dacomitinib, neratinib, afatinib and salts thereof, preferably a combination of an ALK inhibitor that is alectinib or a salt thereof, a TNKS inhibitor selected from AZ6102, G007-LK and salts thereof and a pan-HER inhibitor that is dacomitinib or a salt thereof. In another aspect, it is a combination of an ALK inhibitor selected from alectinib, lorlatinib and salts thereof, a TNKS inhibitor selected from AZ6102, G007-LK, XAV939, 2X-121, JPI-547, RK-582 and salts thereof and a pan-HER inhibitor selected from the group consisting of dacomitinib, neratinib, afatinib and salts thereof, preferably a combination of an ALK inhibitor selected from alectinib, lorlatinib and salts thereof, a TNKS inhibitor selected from AZ6102, G007-LK and salts thereof and a pan-HER inhibitor that is dacomitinib or a salt thereof. In still another aspect, it is a combination of an ALK inhibitor selected from alectinib, brigatinib and salts thereof, a TNKS inhibitor selected from AZ6102, G007-LK, XAV939, 2X-121, JPI-547, RK-582 and salts thereof and a pan-HER inhibitor selected from the group consisting of dacomitinib, neratinib, afatinib and salts thereof, preferably a combination of an ALK inhibitor selected from alectinib, brigatinib and salts thereof, a TNKS inhibitor selected from AZ6102, G007-LK and salts thereof and a pan-HER inhibitor that is dacomitinib or a salt thereof.

Cancer Type

The drug of the present invention is useful for the prevention or treatment of diseases such as various cancers such as leukemia (acute myelocytic leukemia, chronic myelocytic leukemia, acute lymphocytic leukemia, chronic lymphocytic leukemia and the like), malignant lymphoma (Hodgkin's lymphoma, non-Hodgkin's lymphoma and the like), brain tumor, neuroblastoma, glioma, thyroid cancer, myelodysplastic syndrome, head and neck cancer, esophageal cancer, stomach cancer, bowel cancer, colorectal cancer, breast cancer, ovarian cancer, lung cancer, pancreatic cancer, liver cancer, gallbladder cancer, skin cancer, malignant melanoma, kidney cancer, pyeloureteral cancer, bladder cancer, uterine cancer, testicular cancer, and prostate cancer. Furthermore, the compound of the present invention is useful for the prevention or treatment of the invasion and metastasis of solid cancer.

In particular, the drug of the present invention is useful for the prevention or treatment of ALK fusion gene-positive cancer. Examples of the ALK fusion gene-positive cancer include, but are not limited to, non-Hodgkin's lymphoma, neuroblastoma, inflammatory myofibroblastic cytoma, kidney cancer and lung cancer.

In the present invention, being "ALK-fusion gene-positive" means that a fusion gene of ALK and another gene is present. Examples of the other fusion gene include, but are not limited to, echinoderm microtubule-associated protein like protein 4 (EML 4, GeneBank ™ accession number NM_019063), TFG (TRK-fused gene, GeneBank ™ accession number NM_006070).

The method for identifying ALK fusion gene-positive cancer is described in WO2008/127248, Japanese Patent Laid-Open No. 2008-295444, "Guidance for ALK Gene Testing" (Biomarker Committee, the Japan Lung Cancer Society), and the like. As Kits for detecting such a fusion gene, Vysis LSI ALK Break Apart Rearrangement Probe Kit ™ (Abbott) and Histofine ALK iAEP ™ Kit (Nichirei Corporation) are commercially available.

"ALK sensitivity" means having sensitivity to ALK inhibitor. Specifically, it means that when an ALK inhibitor is once applied to a patient with ALK fusion gene-positive cancer or ALK fusion gene-positive cancer cells, a certain level of reducing action or growth suppressive action on ALK fusion gene-positive cancer cells is exhibited as compared to a control such as a solvent or as compared to a state before administration of the ALK inhibitor.

The term "resistant on a temporary basis" means a state of being resistant to an inhibitor on a temporary basis due to short-term exposure to the inhibitor, which is a state in which there is no resistance-related genetic mutation. Here, the resistance-related genetic mutation is, for example, a gatekeeper mutation of an ALK gene.

The "ALK fusion gene-positive cancer resistant to an ALK inhibitor on a temporary basis" means ALK fusion gene-positive cancer which is sensitive to an ALK inhibitor when cells acquired from a subject before the start of treatment in the present invention are cultured in the absence of the ALK inhibitor for a certain period although the ALK fusion gene-positive cancer acquired from the subject before the start of the treatment to the ALK inhibitor is less sensitive to the ALK inhibitor as compared to the same types of cells which have not been exposed to the ALK inhibitor in a known in vivo test using cultures of ALK fusion gene-positive cancer cells acquired from the subject before the start of the treatment in the present invention (for example, Anticancer Drugs. 2022 Feb 1; 33(2): 124-131). The certain period means a period of 6 days or more and 14 days or less, and is preferably at least 9 days and at most 13 days.

Specifically, the ALK fusion gene-positive cancer is such that a subject having ALK fusion gene-positive cancer has received prior treatment with an ALK inhibitor for at least 6 days before the start of the treatment in the present invention, and the cells acquired from the ALK fusion gene-positive cancer acquired from the subject have no resistance-related genetic mutation at the time of starting the treatment in the present invention.

The cell proliferation inhibitory effect of the combination drug of the present invention on ALK fusion gene-positive cancer that is resistant on a temporary basis provides therapeutic advantages such as prevention of resistance acquisition associated with mutation of a resistant gene, and elimination of a necessity of, for example, changing drug holidays and drugs used.

The combination of an ALK inhibitor and a TNKS inhibitor which is used for a drug, a combination, a pharmaceutical composition and a treatment/prevention method for treating/preventing ALK fusion gene-positive cancer in the present invention is specifically a combination of an ALK inhibitor that is alectinib or a salt thereof and a TNKS inhibitor selected from AZ6102, G007-LK, XAV939, 2X-121, JPI-547, RK-582 and salts thereof, where the ALK fusion gene-positive cancer is ALK fusion gene-positive lung cancer, preferably a combination of an ALK inhibitor that is alectinib or a salt thereof and a TNKS inhibitor selected from AZ6102, G007-LK and salts thereof, where the ALK fusion gene-positive cancer is ALK fusion gene-positive lung cancer. In another aspect, it is a combination of an ALK inhibitor selected from alectinib, lorlatinib and salts thereof and a TNKS inhibitor selected from AZ6102, G007-LK, XAV939, 2X-121, JPI-547, RK-582 and salts thereof, where the ALK fusion gene-positive cancer is ALK fusion gene-positive lung cancer, preferably a combination of an ALK inhibitor selected from alectinib, lorlatinib and salts thereof and a TNKS inhibitor selected from AZ6102, G007-LK and salts thereof, where the ALK fusion gene-positive cancer is ALK fusion gene-positive lung cancer. In still another aspect, it is a combination of an ALK inhibitor selected from alectinib, brigatinib and salts thereof and a TNKS inhibitor selected from AZ6102, G007-LK, XAV939, 2X-121, JPI-547, RK-582 and salts thereof, where the ALK fusion gene-positive cancer is ALK fusion gene-positive lung cancer, preferably a combination of an ALK inhibitor selected from alectinib, brigatinib and salts thereof and a TNKS inhibitor selected from AZ6102, G007-LK and salts thereof, where the ALK fusion gene-positive cancer is ALK fusion gene-positive lung cancer. In still another aspect, it is a combination of an ALK inhibitor selected from alectinib, lorlatinib, brigatinib and salts thereof and a TNKS inhibitor selected from AZ6102, G007-LK and salts thereof, where the ALK fusion gene-positive cancer is ALK fusion gene-positive lung cancer.

The combination of an ALK inhibitor and a TNKS inhibitor which is used for a drug, a combination, a pharmaceutical composition and a treatment/prevention method for treating/preventing ALK fusion gene-positive cancer in the present invention is specifically a combination of an ALK inhibitor that is alectinib or a salt thereof and a TNKS inhibitor selected from AZ6102, G007-LK, XAV939, 2X-121, JPI-547, RK-582 and salts thereof, where the ALK fusion gene-positive cancer is ALK fusion gene-positive lung cancer resistant to the ALK inhibitor on a temporary basis, preferably a combination of an ALK inhibitor that is alectinib or a salt thereof and a TNKS inhibitor selected from AZ6102, G007-LK and salts thereof, where the ALK fusion gene-positive cancer is ALK fusion gene-positive lung cancer resistant to the ALK inhibitor on a temporary basis. In another aspect, it is a combination of an ALK inhibitor selected from alectinib, lorlatinib and salts thereof and a TNKS inhibitor selected from AZ6102, G007-LK, XAV939, 2X-121, JPI-547, RK-582 and salts thereof, where the ALK fusion gene-positive cancer is ALK fusion gene-positive lung cancer resistant to the ALK inhibitor on a temporary basis, preferably a combination of an ALK inhibitor selected from alectinib, lorlatinib and salts thereof and a TNKS inhibitor selected from AZ6102, G007-LK and salts thereof, where the ALK fusion gene-positive cancer is ALK fusion gene-positive lung cancer resistant to the ALK inhibitor on a temporary basis. In still another aspect, it is a combination of an ALK inhibitor selected from alectinib, brigatinib and salts thereof and a TNKS inhibitor selected from AZ6102, G007-LK, XAV939, 2X-121, JPI-547, RK-582 and salts thereof, where the ALK fusion gene-positive cancer is ALK fusion gene-positive lung cancer resistant to the ALK inhibitor on a temporary basis, preferably a combination of an ALK inhibitor selected from alectinib, brigatinib and salts thereof and a TNKS inhibitor selected from AZ6102, G007-LK and salts thereof, where the ALK fusion gene-positive cancer is ALK fusion gene-positive lung cancer resistant to the ALK inhibitor on a temporary basis. In still another aspect, it is a combination of an ALK inhibitor selected from alectinib, lorlatinib, brigatinib and salts thereof and a TNKS inhibitor selected from AZ6102, G007-LK and salts thereof, where the ALK fusion gene-positive cancer is ALK fusion gene-positive lung cancer resistant to the ALK inhibitor on a temporary basis.

In addition, the combination of an ALK inhibitor, a TNKS inhibitor and a pan-HER inhibitor which is used for a drug, a combination, a pharmaceutical composition and a treatment/prevention method for treating/preventing ALK fusion gene-positive cancer in the present invention is a combination of an ALK inhibitor that is alectinib or a salt thereof, a TNKS inhibitor selected from AZ6102, G007-LK, XAV939, 2X-121, JPI-547, RK-582 and salts thereof and a pan-HER inhibitor selected from the group consisting of dacomitinib, neratinib, afatinib and salts thereof, where the ALK fusion gene-positive cancer is ALK fusion gene-positive lung cancer, preferably a combination of an ALK inhibitor that is alectinib or a salt thereof, a TNKS inhibitor selected from AZ6102, G007-LK and salts thereof and a pan-HER inhibitor that is dacomitinib or a salt thereof, where the ALK fusion gene-positive cancer is ALK fusion gene-positive lung cancer. In another aspect, it is a combination of an ALK inhibitor selected from alectinib, lorlatinib and salts thereof, a TNKS inhibitor selected from AZ6102, G007-LK, XAV939, 2X-121, JPI-547, RK-582 and salts thereof and a pan-HER inhibitor selected from the group consisting of dacomitinib, neratinib, afatinib and salts thereof, where the ALK fusion gene-positive cancer is ALK fusion gene-positive lung cancer, preferably a combination of an ALK inhibitor selected from alectinib, lorlatinib and salts thereof, a TNKS inhibitor selected from AZ6102, G007-LK and salts thereof and a pan-HER inhibitor that is dacomitinib or a salt thereof,

where the ALK fusion gene-positive cancer is ALK fusion gene-positive lung cancer. In still another aspect, it is a combination of an ALK inhibitor selected from alectinib, brigatinib and salts thereof, a TNKS inhibitor selected from AZ6102, G007-LK, XAV939, 2X-121, JPI-547, RK-582 and salts thereof and a pan-HER inhibitor selected from the group consisting of dacomitinib, neratinib, afatinib and salts thereof, where the ALK fusion gene-positive cancer is ALK fusion gene-positive lung cancer, preferably a combination of an ALK inhibitor selected from alectinib, brigatinib and salts thereof, a TNKS inhibitor selected from AZ6102, G007-LK and salts thereof and a pan-HER inhibitor that is dacomitinib or a salt thereof, where the ALK fusion gene-positive cancer is ALK fusion gene-positive lung cancer.

In another aspect of the present invention, there is provided a method for enhancing the therapeutic effect of an ALK inhibitor in treatment of ALK fusion gene-positive cancer with a drug comprising the ALK inhibitor and a TNKS inhibitor in combination, or a triple-drug combination of an ALK inhibitor, a TNKS inhibitor and a pan-HER inhibitor. Here, enhancing the therapeutic effect means either that the treatment success rate increases as compared to the case of single-agent administration; the amount of an ALK inhibitor administered in treatment with a combination drug is reduced as compared to the case of single administration; a therapeutic effect is shown in more severe cases, for example, in patients with prior treatment failure or exacerbation; the duration of treatment with the ALK inhibitor is shorter than the expected duration of treatment with the compound alone or the duration of treatment planned before starting the treatment by combined administration, due to the disappearance of the cancer or the like; or that the duration of treatment including the ALK inhibitor and/or other maintenance therapy is prolonged by the suppression of disease progression. In a preferred aspect of the present invention, the combination drug and the like according to the present invention have a synergic effect. Here, the "synergic effect" means that the effect of a combination of two drugs is greater than the sum of the effects of the individual drugs. In another embodiment, the combination drug and the like according to the present invention have an additive effect. Here, the "additive effect" of two compounds means that the effect of a combination of two or three drugs is the sum of the effects of the individual drugs. In addition, in another aspect of the present invention, there is provided a method of prolonging the progression-free survival (PFS) in a subject, comprising administering an effective amount of an ALK inhibitor and an effective amount of a TNKS inhibitor in combination. In addition, in another aspect of the present invention, there is provided a method of using an ALK inhibitor or a TNKS inhibitor for producing a pharmaceutical composition for treating or preventing target cancer, comprising the ALK inhibitor and the TNKS inhibitor as active ingredients.

In the present invention, "progression-free survival (PFS)" refers to the time from treatment (or randomization) to first disease progression or death. In one aspect of the present invention, PFS can be assessed by the Response Evaluation Criteria in Solid Tumors (RECIST). In one aspect of the present invention, PFS can be assessed by CA-125 levels as a determinant of progression.

In the present invention, specific examples of "prolonging progression-free survival" include the use of an ALK inhibitor and a TNKS inhibitor in combination to prolong PFS compared to the PFS when the ALK inhibitor or the TNKS inhibitor is administered as a single agent at the same dose used when in combination.

In the present invention, comprising an ALK inhibitor and/or a TNKS inhibitor as an active ingredient means comprising a an ALK inhibitor and/or a TNKS inhibitor as a main active ingredient, and does not limit the content of the ALK inhibitor and/or the TNKS inhibitor. In addition, the term "treatment" means that the administration of the drug according to the present invention to a subject causes the death of cancer cells or a decrease in the number, the weight and/or the volume of those cells, the suppression of the growth of cancer cells after a certain period from the start of the administration (that is, a decrease in the number, the weight and/or the volume of cancer cells after a certain period from the start of the administration as compared to the number, the weight and/or the volume of non-treated cancer cells left for the same period), the suppression of the metastasis of cancer cells, the extension of progression-free survival (PFS) as compared to the case of monotherapy, or the improvement of various symptoms caused by target cancer. In addition, the word "prevention" means either preventing an increase in the number of cancer cells that had been reduced from when they grow again, or preventing the regrowth of cancer cells whose growth had been suppressed.

In the present invention, the "effective amount" means the daily dose of each inhibitor when an ALK inhibitor and a TNKS inhibitor are administered in combination. The dose in the present invention may be the same as the dose when each inhibitor is used alone, or it may be a lower dose than the dose when each inhibitor is used alone. Alternatively, the effective amount in the present invention is the same as the dose when one is used alone, and it may be a lower dose than the dose when the other is used alone.

Another aspect of the present invention includes an agent for inhibiting the growth of ALK fusion gene-positive cancer resistant to an ALK inhibitor on a temporary basis, comprising an ALK inhibitor and a TNKS inhibitor in combination; an agent for inhibiting the growth of ALK fusion gene-positive cancer resistant to an ALK inhibitor on a temporary basis, comprising an ALK inhibitor as an active ingredient, which is used in combination with a TNKS inhibitor; an agent for inhibiting the growth of ALK fusion gene-positive cancer resistant to an ALK inhibitor on a temporary basis, comprising a TNKS inhibitor as an active ingredient, which is used in combination with an ALK inhibitor; and a method for inhibiting the growth of ALK fusion gene-positive cancer resistant to an ALK inhibitor on a temporary basis, comprising administering an effective amount of an ALK inhibitor and an effective amount of a TNKS inhibitor in combination to a subject.

The drug or method for inhibiting the growth of ALK fusion gene-positive cancer resistant to an ALK inhibitor on a temporary basis refers to an agent or method capable of inhibiting the growth by using an ALK inhibitor and a TNKS inhibitor in combination on ALK fusion gene-positive cancer having resistance on a temporary basis due to prior treatment with the ALK inhibitor or the like.

Here, inhibiting the growth of ALK fusion gene-positive cancer means that a subject having ALK fusion gene-positive cancer is treated with an ALK inhibitor and a TNKS inhibitor in combination to reduce or shrink the weight and/or the volume of ALK fusion gene-positive cancer after the treatment according to the present invention as compared to the weight and/or the volume of ALK fusion gene-positive cancer after the same period which is predicted from the growth rate before the start of the treatment. Alternatively, it means that the averages of the weights and/or volumes of ALK fusion gene-positive cancer after the treatment of a plurality of subjects according to the present invention for a certain period are reduced or shrunk, preferably significantly reduced or shrunk, as compared to the averages of the weights and/or volumes of ALK fusion gene-positive cancer after the same period in a plurality of non-treated subjects. Alternatively, it means that the averages of the weights and/or volumes of ALK fusion gene-positive cancer after the treatment of a plurality of subjects according to the present invention for a certain period are reduced or shrunk, preferably significantly reduced or shrunk, as compared to the averages of the weights and/or volumes of ALK fusion gene-positive cancer after the same period in a plurality of subjects that have received one of an ALK inhibitor and a TNKS inhibitor.

The present invention also includes a method for shrinking ALK fusion gene-positive cancer. Here, "shrinking" ALK fusion gene-positive cancer means that a subject having ALK fusion gene-positive cancer is treated with an ALK inhibitor and a TNKS inhibitor in combination to reduce or shrink the weight and/or the volume of ALK fusion gene-positive cancer after the treatment according to the present invention as compared to the weight and/or the volume of ALK fusion gene-positive cancer before the start of the treatment. Alternatively, it means that the averages of the weights and/or volumes of ALK fusion gene-positive cancer after the treatment of a plurality of subjects according to the present invention for a certain period are reduced or shrunk, preferably significantly reduced or shrunk, as compared to the averages of the weights and/or volumes of ALK fusion gene-positive cancer before the start of the treatment.

Still another aspect of the present invention includes an agent for inhibiting the growth of ALK fusion gene-positive cancer cells resistant to an ALK inhibitor on a temporary basis, comprising an ALK inhibitor and a TNKS inhibitor in combination; an agent for inhibiting the growth of ALK fusion gene-positive cancer cells resistant to an ALK inhibitor on a temporary basis, comprising an ALK inhibitor as an active ingredient, which is used in combination with a TNKS inhibitor; an agent for inhibiting the growth of ALK fusion gene-positive cancer cells resistant to an ALK inhibitor on a temporary basis, comprising a TNKS inhibitor as an active ingredient, which is used in combination with an ALK inhibitor; and a method for inhibiting the growth of ALK fusion gene-positive cancer cells resistant to an ALK inhibitor on a temporary basis, comprising administering an effective amount of an ALK inhibitor and an effective amount of a TNKS inhibitor in combination to a subject. Here, "inhibiting the growth" of ALK fusion gene-positive cancer cells means that ALK fusion gene-positive cancer cells are treated with an ALK inhibitor and a TNKS inhibitor in combination to reduce or shrink the weight and/or the volume of ALK fusion gene-positive cancer after the treatment according to the present invention as compared to the percentage of proliferation of untreated ALK fusion gene-positive cancer cells after the same period. Alternatively, it means that the average of the percentages of proliferation of ALK fusion gene-positive cancer cells after the treatment according to the present invention for a certain period are reduced, preferably significantly reduced, as compared to the average of the percentages of proliferation after the same period in ALK fusion gene-positive cancer cells treated with one of an ALK inhibitor and a TNKS inhibitor.

In the present invention, the "subject" means, but is not limited to, a mammal including a human or a non-human mammal, for example, a cow, a horse, a dog, a sheep, or a cat, that is the subject of administration of the drug of the present invention or requires the administration of the drug of the present invention. Preferably, the subject is human. The subject includes a patient (including human and non-human mammal). Specifically, these are patients having, or humans or non-human mammals likely to have, various cancers such as leukemia (acute myelocytic leukemia, chronic myelocytic leukemia, acute lymphocytic leukemia, chronic lymphocytic leukemia and the like), malignant lymphoma (Hodgkin's lymphoma, non-Hodgkin's lymphoma and the like), brain tumor, neuroblastoma, glioma, thyroid cancer, myelodysplastic syndrome, head and neck cancer, esophageal cancer, stomach cancer, bowel cancer, colorectal cancer, breast cancer, ovarian cancer, lung cancer, pancreatic cancer, liver cancer, gallbladder cancer, skin cancer, malignant melanoma, renal cancer, pyeloureteral cancer, bladder cancer, uterine cancer, testicular cancer, prostate cancer, and the tumors metastasized from these tumors.

Product

As another aspect of the present invention, a product is provided. In one embodiment, a product is provided for treating or preventing target cancer in a subject, and comprises: (a) (1) a preparation comprising a TNKS inhibitor, (2) a container, and (3) an instruction or label indicating that the TNKS inhibitor and at least one ALK inhibitor are administered in combination to a subject for treating target cancer in the subject; or (b) (1) a preparation comprising an ALK inhibitor, (2) a

container, and (3) an instruction or label indicating that the ALK inhibitor and at least one TNKS inhibitor are administered in combination to a subject for treating target cancer in the subject.

In another embodiment of the present invention, (c1) (1) a preparation comprising an ALK inhibitor, (2) a container, and (3) an instruction or label indicating that preparations comprising the ALK inhibitor, at least one TNKS inhibitor and at least one pan-HER inhibitor are administered in combination to a subject for treating target cancer in the subject; or (c2) (1) a preparation comprising a TNKS inhibitor, (2) a container, and (3) an instruction or label indicating that preparations comprising the TNKS inhibitor, at least one ALK inhibitor and at least one pan-HER inhibitor are administered in combination to a subject for treating target cancer in the subject are included. Alternatively, (c3) (1) a preparation comprising a pan-HER inhibitor, (2) a container, and (3) an instruction or label indicating that the pan-HER inhibitor, at least one TNKS inhibitor and at least one ALK inhibitor are administered in combination to a subject for treating target cancer in the subject are included.

The product comprises a container comprising a preparation comprising a TNKS inhibitor, a pan-HER inhibitor or an ALK inhibitor.

The product may further comprise a label or instruction on or accompanying the container.

In the present invention, the "instruction" means a document normally included in the commercial packaging of a preparation, which includes information on the indications, usage, dose, administration, contraindications and/or warnings regarding the use of the preparation. The "label" means a sheet-shaped article containing the indication of the product name, dosage, dosage form, indications, and the like of a preparation comprising a TNKS inhibitor or an ALK inhibitor, which is affixed directly to the container.

The label or instruction indicates the indications of the preparation, i.e., that it is to be used for the treatment of target cancer and the like. In one embodiment, the label or instruction indicates that the preparation can be used to treat target cancer, and the like. The label or instruction may also indicate that the preparation can be used to treat other disorders.

Examples of suitable containers include PTP, a bottle, a vial, a syringe, and a blister pack. The container may be formed from a variety of materials such as glass or plastic. These containers may be further packaged in a paper outer box on which the content of the above label and the like are printed.

[Examples]

All patents and references expressly cited herein are incorporated herein by reference in their entirety.

Hereinafter, the present invention will be specifically described with reference to the descriptions of Examples, but the present invention is not to be construed as limited by these descriptions.

The drugs, reagents and cells used in Examples are as follows.

Alectinib was synthesized by Chugai Pharmaceutical Co., Ltd. For the compound library, 3114 compounds obtained by excluding poisonous drugs from 3116 compounds of Anti-cancer compound library (catalog No. L2100) from TargetMol Chemicals Inc. were used. AZ6102, G007-LK, XAV939 and Dacomitinib used for *in vitro* assays were obtained from Selleck Chemicals LLC. All the drugs were dissolved in dimethyl sulfoxide (DMSO: Sigma-Aldrich) for *in vitro* assays. G007-LK and Dacomitinib used for *in vivo* assays were obtained from ChemScene LLC and TargetMol Chemicals Inc., respectively. Alectinib was dissolved in 6% captisol for in vivo assays. G007-LK and Dacomitinib were dissolved in 5% DMSO, 30% PEG 300 and 5% Tween 80.

The human cancer cell strain 15cl used in Examples is a cell strain established by performing single cell cloning on the ALK fusion gene-positive lung cancer patient-derived human cancer cell strain 1510B3N (EML4-ALK variant 1) established in Japanese Foundation for Cancer Research under the ALCURE observational study (UMIN000038934) (source: Japanese Foundation for Cancer Research, The Cancer Chemotherapy Center). The culture medium for 15cl is 15% FBS in RPMI 1640/Ham's F-12. The cells were cultured at 37°C under 5% $CO_2$.

Example 1

The 15cl cells were treated with alectinib for 9 days to prepare drug tolerant persister (DTP) cells (15DT) having transient resistance to alectinib. The DTP cell means a cell that has acquired drug resistance without genetic mutation through a certain period of drug exposure. The number of viable cells 6 days after exposure of each of 15cl and 15DT to the compound library was measured. Exposure to each compound was performed at a final concentration of 100 nM. Figure 1 shows the results of analyzing the percentage of cell proliferation after exposure to each compound. In Figure 1, the vertical axis indicates the value calculated by the following expression 1, and the horizontal axis indicates each compound. The percentage of cell proliferation of each compound in expression 1 was calculated by a known method (Mol Cancer Ther. 2021 Jun; 20(6): 1133-1141.). It is indicated that the proliferation inhibitory effect of the compound increases in DTP cells when the ratio of cell proliferation (15DT/15cl) in Figure 1 is less than 1, and in contrary, the proliferation inhibitory effect decreases when the ratio of cell proliferation is more than 1. In Figure 1, only compounds targeting TNKS (tankyrase) in

L2100 from TargetMol are highlighted.

[Expression 1]

$$\text{Ratio of cell proliferation}\left(\frac{15DT}{15cl}\right)=\frac{\text{percentage of cell proliferation under each compound in 15DT}}{\text{percentage of cell proliferation under each compound in 15cl}}$$

As a result, it was indicated that four of the five compounds targeting TNKS in L2100 showed a value of less than 1, and their proliferation inhibitory effects increased in DTP cells.

For the purpose of verifying the results of screening with the compound library, 15cl and 15DT were seeded on a 384-well plate, and the number of viable cells 6 days after exposure to each of alectinib as an ALK inhibitor and AZ6102, G007-LK and XAV939 as TNKS inhibitors at a concentration shown in Figure 2 was measured. Figure 2 shows the results of calculating the percentage of cell proliferation with respect to the number of agent-non-treated cells. In Figure 2, the vertical axis indicates the percentage of cell proliferation, and the horizontal axis indicates the concentration of the compound.

As a result, it was indicated that 15DT was less sensitive to alectinib than 15 cl. In addition, it was indicated that AZ6102 and G007-LK whose proliferation inhibitory effect increased in 15DT in screening with the compound library had no proliferation inhibitory effect in 15cl, but had a concentration-dependent proliferation inhibitory effect in 15DT, and their proliferation inhibitory effects increased in 15DT. In addition, it was indicated that for XAV939, the proliferation inhibitory effect did not increase in 15DT in the compound library at a final concentration of 100 nM, but at a higher concentration, the proliferation inhibitory effect increased in 15DT as compared to that in 15cl as shown in Figure 2.

Example 2

The 15cl was seeded on a 6-well plate, a drug was added thereto, the cells were cultured for a time shown in Figure 3, and proteins were purified from the cells. Figure 3 shows the results of observing the expression levels of each protein using the capillary electrophoresis protein analysis system Sally Sue (ProteinSimple). Note that, in Figure 3, ALC indicates alectinib, - indicates agent non-treated, and h and d indicate hour and day, respectively. As the primary antibody, antibodies to phospho (p)-β-catenin (Ser675), non-p-β-catenin (Ser33/37/Thr41), β-catenin, Survivin, c-Myc/N-Myc and Actinβ (ACTB) (Cell Signaling Technology), an antibody to Axin1 (Thermo Fisher Scientific), and an antibody to Axin2 (abcam) were used. It is known that Ser675 for β-catenin is phosphorylated at the time of activation, and Ser33/37/Thr41 is phosphorylated at the time of deactivation.

As a result, it was indicated that as compared to agent non-treated, the amount of active β-catenin (p-β-catenin and non-p-β-catenin) increased 24 hours to 9 days after alectinib treatment. In addition, it was indicated that the amount of Axin1 and Axin2 involved in degradation of β-catenin and targeted to be degraded by tankyrase continuously decreased by alectinib treatment. In addition, it was indicated that the amount of Survivin and c-Myc/N-Myc, the transcription of which is activated by β-catenin decreased immediately after alectinib treatment, but increased with an increase in the amount of active β-catenin.

Active β-catenin is known to move from the cytoplasm to the nucleus and activate the transcription of a target gene. Therefore, for the purpose of observing the amount of β-catenin localized in the nucleus, 15 cl and 15DT were seeded in a flask, a drug was added, the cells were cultured for a time shown in Figure 4, a nuclear fraction and a cytoplasmic fraction were fractionated using Nuclear/Cytosolic Fractionation Kit (Cell Biolabs Inc.), and proteins were purified. Figure 4 shows the results of observing the expression levels of the proteins using Sally Sue. Note that, in Figure 4, ALC indicates alectinib, NT indicates agent non-treated, h indicates hour, and Cyt and Nuc indicate a cytoplasmic fraction and a nuclear fraction, respectively. As the primary antibody, antibodies to β-catenin and β-tubulin (Cell Signaling Technology) and an antibody to Lamin A/C (abcam) were used. Note that β-tubulin and Lamin A/C were used as marker proteins for the cytoplasmic fraction and the nuclear fraction, respectively.

As a result, it was indicated that the amount of β-catenin in the nucleus increased in treatment with alectinib for 24 hours as compared to agent non-treated, and further increased in 15DT, which suggested that movement of β-catenin to the nucleus was increased by alectinib treatment.

From the above, it was suggested that alectinib treatment activated β-catenin.

Example 3

Whether use of an ALK inhibitor and a TNKS inhibitor in combination enhanced the cell proliferation inhibitory

effect of the ALK inhibitor in 15cl was examined. The 15cl was seeded on a 384-well plate, and the number of viable cells 6 days after exposure to alectinib, lorlatinib or brigatinib as an ALK inhibitor and AZ6102 or G007-LK as a TNKS inhibitor at a concentration shown in Figure 5 was measured. Figure 5 shows the results of calculating the percentage of cell proliferation with respect to the number of agent-non-treated cells. In Figure 5, the vertical axis indicates the percentage of cell proliferation, the horizontal axis indicates the concentration of the compound, ALC indicates alectinib, LOR indicates lorlatinib, and BRG indicates brigatinib.

As a result, it was indicated that the TNKS inhibitors as single agents did not exhibit a proliferation inhibitory effect, but use of the TNKS inhibitor and the ALK inhibitor in combination enhanced the cell proliferation inhibitory effect of the ALK inhibitor, which suggested that the ALK inhibitor and the TNKS inhibitor exhibited a synergic effect.

Example 4

The 15cl was seeded on a 6-well plate, a drug was added thereto, the cells were cultured for 48 hours, and proteins were purified from the cells. Figure 6 shows the results of observing the expression levels of the proteins using Sally Sue. Note that, in Figure 6, ALC indicates alectinib, AZ indicates AZ6102, - indicates agent non-treated, and + indicates agent treated. As the primary antibody, antibodies to phospho (p)-β-catenin (Ser675), non-p-β-catenin (Ser33/37/Thr41), β-catenin, Survivin, c-Myc/N-Myc and Actinβ (ACTB) (Cell Signaling Technology), and an antibody to Axin1 (Thermo Fisher Scientific) were used. Note that it is known that c-Myc/N-Myc is involved in cell proliferation and Survivin is involved in anti-apoptosis.

As a result, it was indicated that the amount of active β-catenin which had been increased by alectinib treatment was reduced by treatment with the TNKS inhibitor. In addition, it was indicated that the use in combination reduced the amount of c-Myc/N-Myc and Survivin, the transcription of which is activated by β-catenin.

Example 5

Whether a triple-drug combination of an ALK inhibitor, a TNKS inhibitor and a pan-HER inhibitor enhanced the cell proliferation inhibitory effect as compared to a double-drug combination of an ALK inhibitor and a TNKS inhibitor or an ALK inhibitor and a pan-HER inhibitor in 15 cl was examined. The 15cl was seeded on a 384-well plate, and the number of viable cells 6 days after exposure to alectinib as an ALK inhibitor, AZ6102 as a TNKS inhibitor and dacomitinib as a pan-HER inhibitor at a concentration shown in Figure 7 was measured. Figure 7 shows the results of calculating the percentage of cell proliferation with respect to the number of agent-non-treated cells. In Figure 7, the vertical axis indicates the percentage of cell proliferation, the horizontal axis indicates the concentration of the compound, ALC indicates alectinib, Daco indicates dacomitinib, and AZ indicates AZ6102.

As a result, it was indicated that a double-drug combination of a TNKS inhibitor or a pan-HER inhibitor and an ALK inhibitor enhanced the cell proliferation inhibitory effect as compared to the ALK inhibitor as a single agent, and a triple-drug combination of these inhibitors further enhanced the cell proliferation inhibitory effect, which suggested exhibition of a synergic effect.

Example 6

Alectinib (2 mg/kg, oral administration once daily for 14 days) and G007-LK (10 mg/kg and 50 mg/kg, oral administration once daily for 14 days) were used in combination on SCID mice (CLEA Japan, Inc., male) transplanted with 15cl at $5 \times 10^6$ cells per mouse. Figure 8 shows the tumor volume (mean + standard deviation) and the relative body weight (mean + standard deviation) for each administration group. Alectinib (2 mg/kg, oral administration once daily for 14 days), G007-LK (10 mg/kg, oral administration once daily for 14 days) and dacomitinib (10 mg/kg, oral administration once daily for 14 days) were used in combination, and Figure 9 shows the volume (mean + standard deviation) and the relative body weight (mean + standard deviation) for each administration group.

Note that, in Figures 8 and 9, the horizontal axis indicates the number of days elapsed when the first day of agent administration is set as 1, the vertical axis indicates the tumor volume or the relative body weight, Vehicle indicates the solvent administration control group, ALC indicates alectinib, and Daco indicates dacomitinib. Figure 8 shows the results for n = 6 per group, and Figure 9 shows the results for n = 8 per group.

As a result, in Figure 8, the alectinib and G007-LK combination administration group showed a higher anti-tumor effect than each single agent administration group. On day 15 after the start of agent administration, the alectinib and 10 mg/kg G007-LK combination group showed statistically significantly higher anti-tumor effect than the solvent administration group and the 10 mg/kg G007-LK single agent administration group, and the alectinib and 50 mg/kg G007-LK combination group showed statistically significantly higher anti-tumor effect than the solvent administration group and the 50 mg/kg G007-LK single agent administration group. No weight loss was observed in either group compared to the solvent administration group.

In addition, in Figure 9, the alectinib, G007-LK and dacomitinib triple-drug combination administration group showed a higher anti-tumor effect than the alectinib and G007-LK double-drug combination administration group and the alectinib and dacomitinib double-drug combination administration group. On day 15 after the agent administration, triple-drug combination administration group showed a statistically significantly higher anti-tumor effect than the double-drug combination administration control group. No weight loss was observed in either group.

[Industrial Applicability]

The combination drug of the present invention is useful for the prevention or treatment of ALK fusion gene-positive cancer and the like. In addition, the combination drug of the present invention is useful as an agent for suppressing the growth of ALK fusion gene-positive cancer which is resistant on a temporary basis.

## Claims

1. A drug for treating or preventing ALK fusion gene-positive cancer, comprising an ALK inhibitor and a TNKS inhibitor in combination.

2. The drug according to claim 1, further comprising a pan-HER inhibitor in combination.

3. A drug for treating or preventing ALK fusion gene-positive cancer, comprising an ALK inhibitor as an active ingredient, which is used in combination with a TNKS inhibitor.

4. The drug according to claim 1 or 3, wherein the ALK inhibitor is used concurrently with the TNKS inhibitor, or before the start of administration of the TNKS inhibitor.

5. A drug for treating or preventing ALK fusion gene-positive cancer, comprising a TNKS inhibitor as an active ingredient, which is used in combination with an ALK inhibitor.

6. The drug according to claim 5, wherein the TNKS inhibitor is used concurrently with the ALK inhibitor, or after the start of administration of the ALK inhibitor.

7. The drug according to any one of claims 3, 5 and 6, further comprising a pan-HER inhibitor in combination.

8. The drug according to any one of claims 1 to 3, 5 and 6, wherein the ALK inhibitor is selected from the group consisting of crizotinib, alectinib, ceritinib, lorlatinib, brigatinib and salts thereof.

9. The drug according to any one of claims 1 to 3, 5 and 6, wherein the TNKS inhibitor is selected from the group consisting of AZ6102, G007-LK, XAV939, 2X-121, JPI-547, RK-582 and salts thereof.

10. The drug according to any one of claims 1 to 3, 5 and 6, wherein the pan-HER inhibitor is selected from the group consisting of dacomitinib, neratinib, afatinib and salts thereof.

11. The drug according to any one of claims 1 to 3, 5 and 6, wherein the ALK fusion gene-positive cancer is selected from the group consisting of non-Hodgkin's lymphoma, neuroblastoma, inflammatory myofibroblastic cytoma, kidney cancer and lung cancer.

12. The drug according to any one of claims 1 to 3, 5 and 6, wherein the ALK fusion gene-positive cancer has not received prior treatment with an ALK inhibitor.

13. The drug according to any one of claims 1 to 3, 5 and 6, wherein the ALK fusion gene-positive cancer is sensitive to ALK.

14. The drug according to any one of claims 1 to 3, 5 and 6, wherein the ALK inhibitor is administered simultaneously with the TNKS inhibitor.

15. A method for treating or preventing ALK fusion gene-positive cancer, comprising administering to a subject an effective amount of an ALK inhibitor and an effective amount of a TNKS inhibitor in combination.

**16.** The method according to claim 15, further comprising administering a pan-HER inhibitor in combination.

**17.** An agent for inhibiting the growth of ALK fusion gene-positive cancer resistant to an ALK inhibitor on a temporary basis, comprising an ALK inhibitor and a TNKS inhibitor in combination.

**18.** The growth inhibiting agent according to claim 17, further comprising a pan-HER inhibitor in combination.

**19.** A product comprising: (1) a preparation comprising an ALK inhibitor, (2) a container, and (3) an instruction or label indicating that the ALK inhibitor is to be administered to a subject in combination with at least one TNKS inhibitor for treating ALK fusion gene-positive cancer.

**20.** The product according to claim 19, comprising an instruction or label indicating that a pan-HER inhibitor is to be further administered in combination.

**21.** A combination of an ALK inhibitor and a TNKS inhibitor or use thereof for producing a drug for treating or preventing ALK fusion gene-positive cancer.

Figure 1

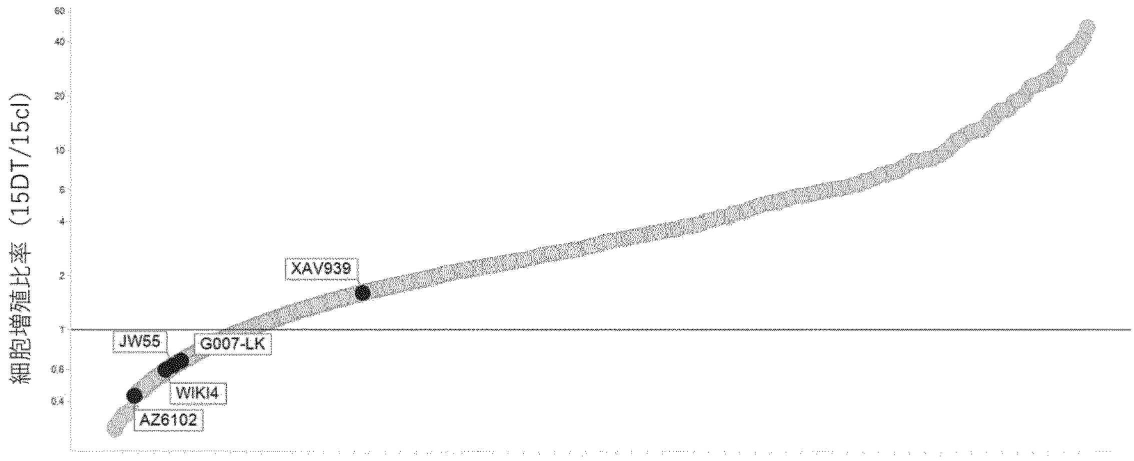


Figure 2

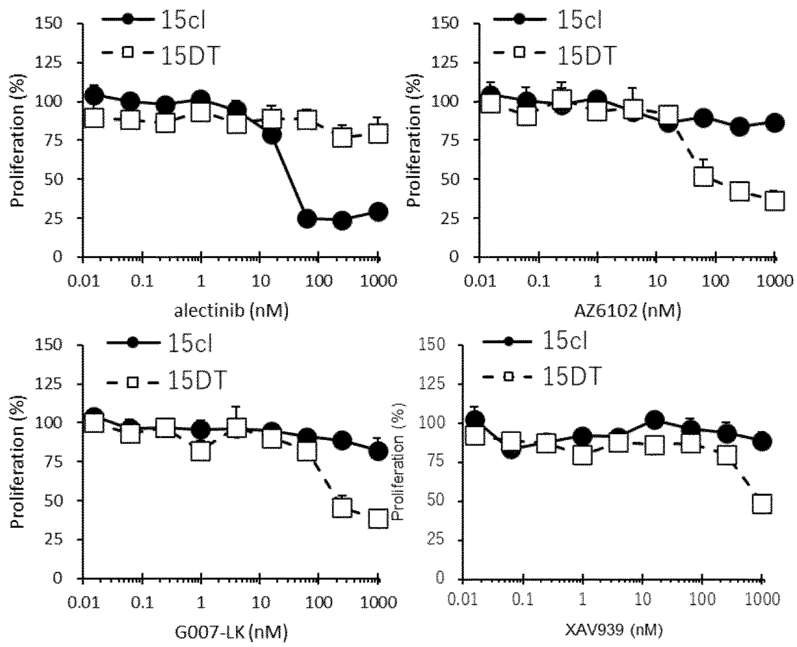

Figure 3

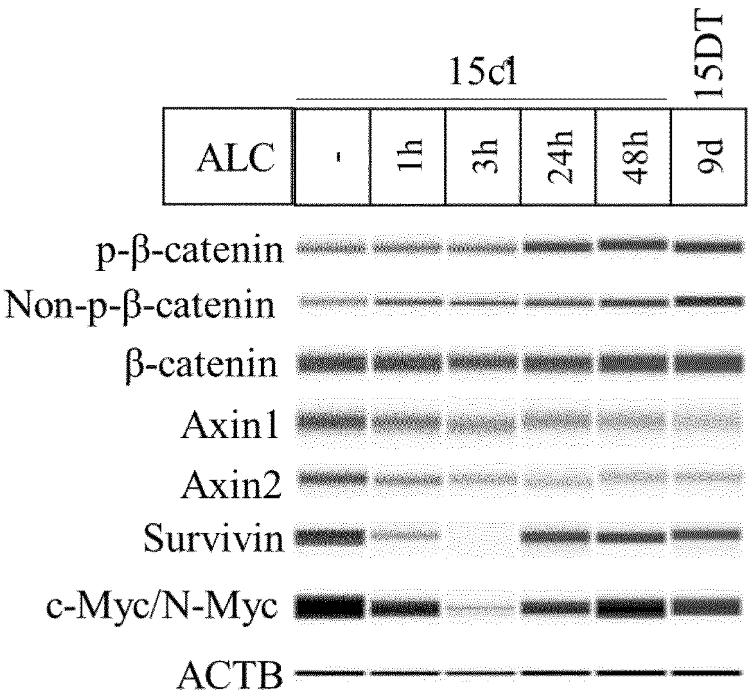


Figure 4

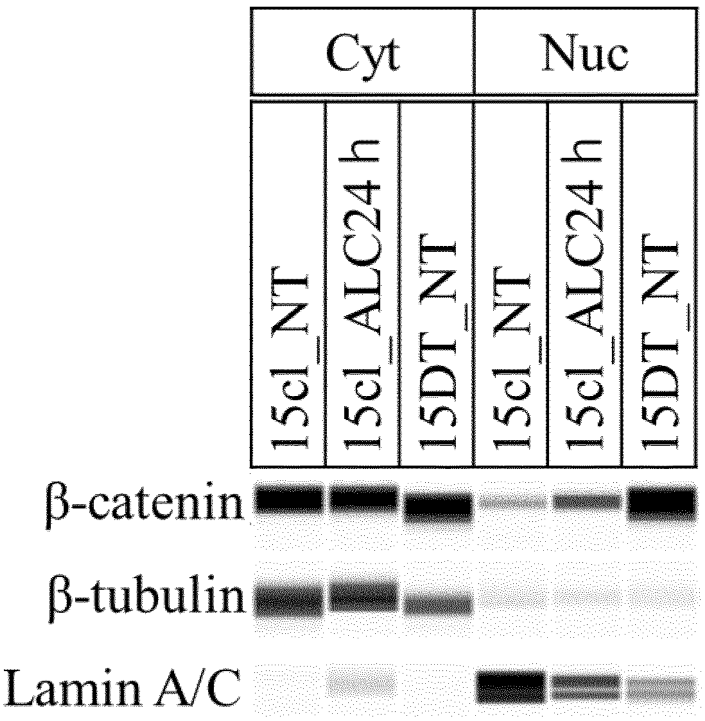

Figure 5

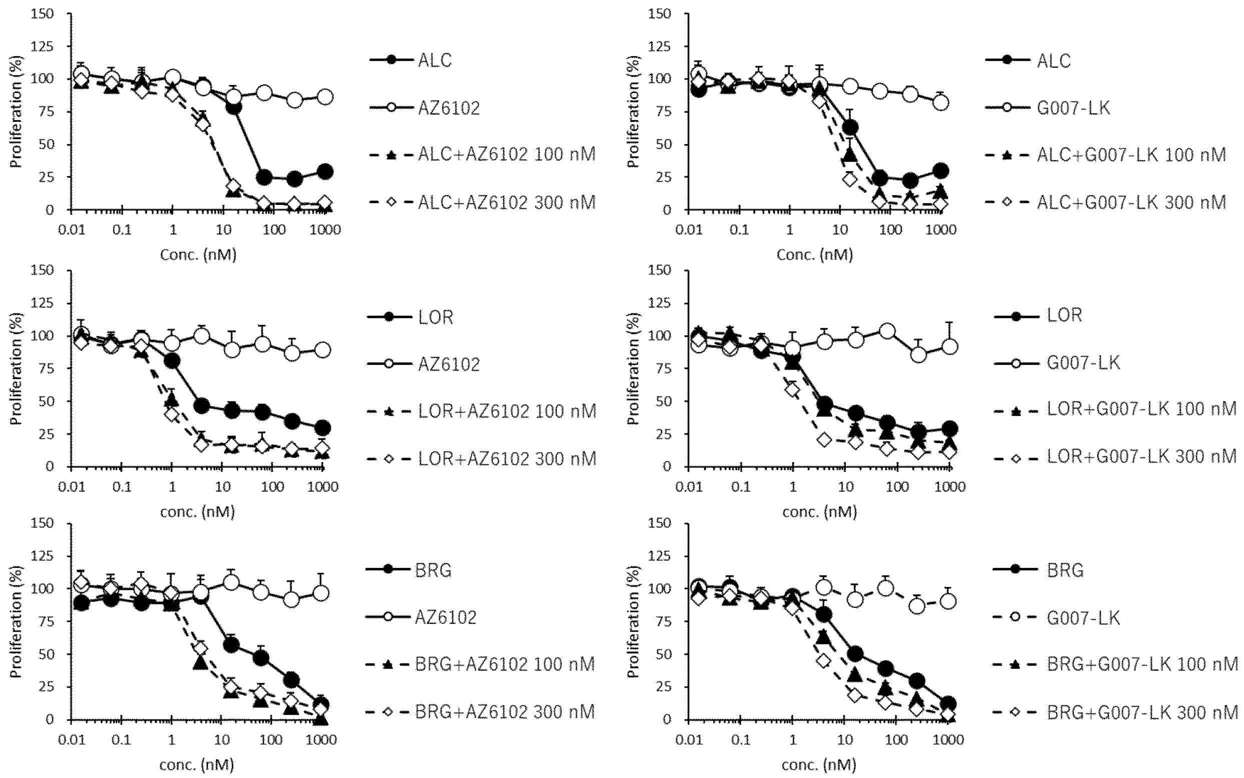


Figure 6

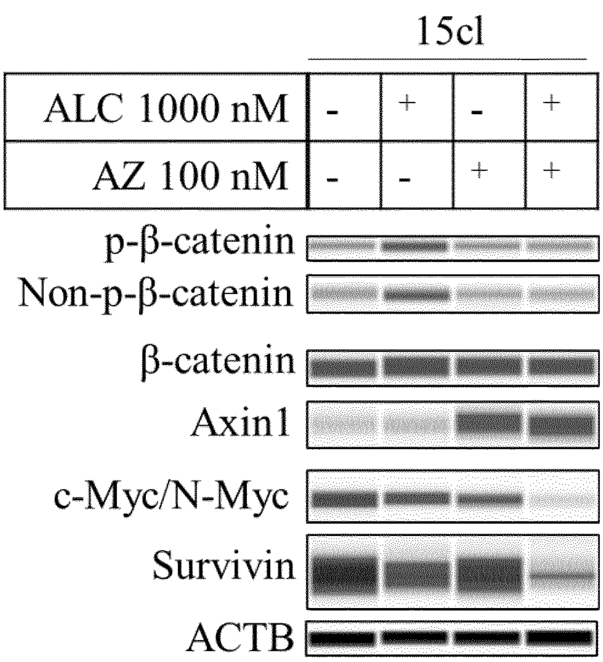

Figure 7

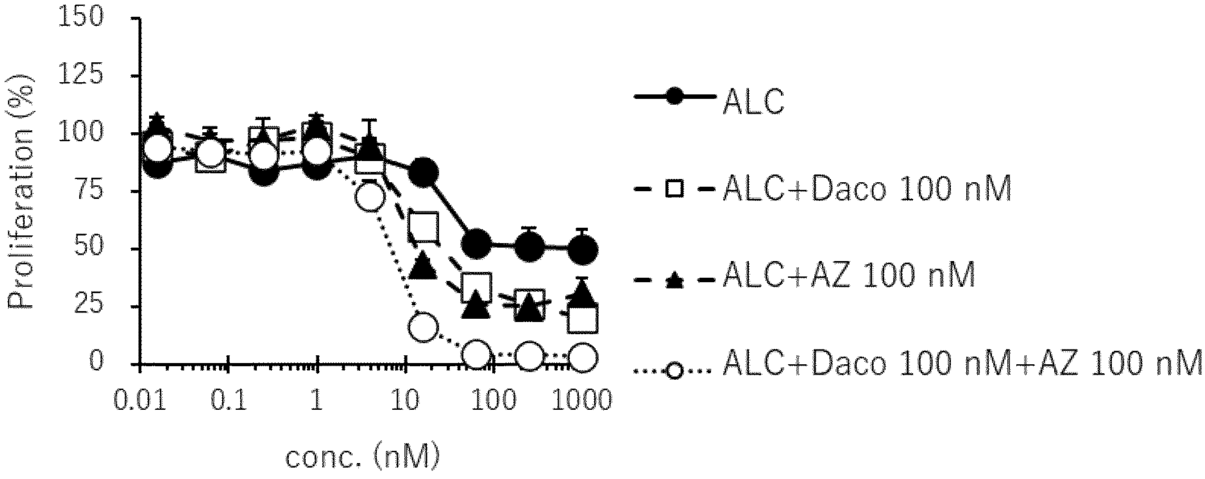


Figure 8

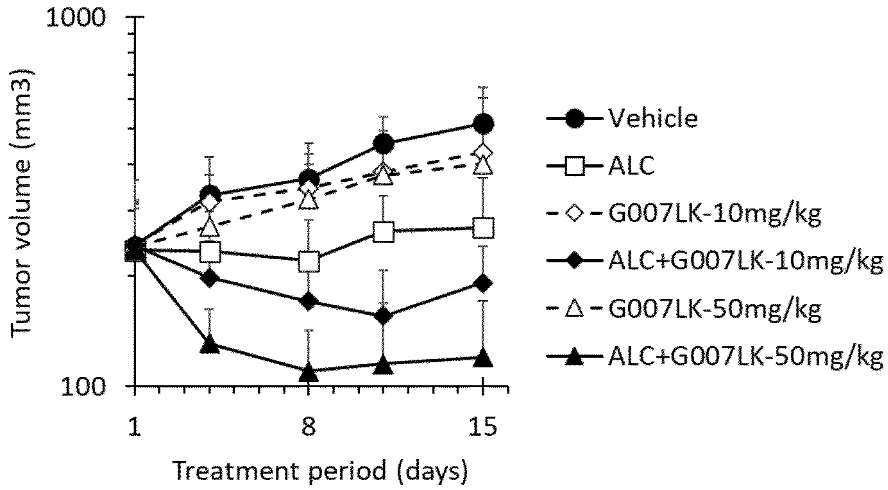


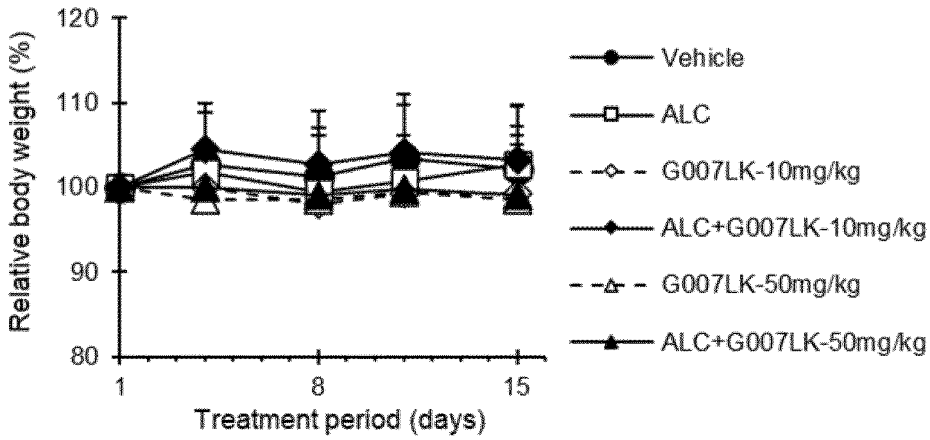

Figure 9

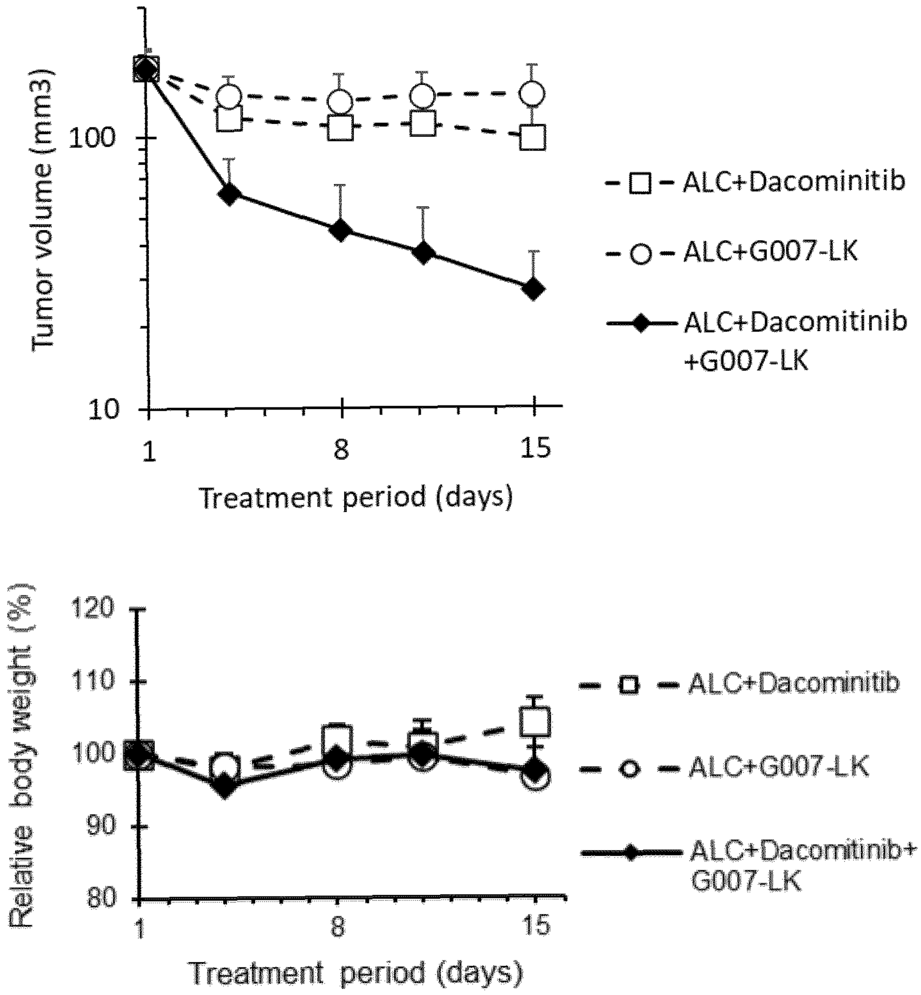
Tumor volume (mm3)
100
10
1
8
15
Treatment period (days)
ALC+Dacominitib
ALC+G007-LK
ALC+Dacomitinib
+G007-LK
Relative body weight (%)
120
110
100
90
80
1
8
15
Treatment period (days)
ALC+Dacominitib
ALC+G007-LK
ALC+Dacomitinib+
G007-LK

## INTERNATIONAL SEARCH REPORT

International application No.
**PCT/JP2023/031120**

**A. CLASSIFICATION OF SUBJECT MATTER**

***A61K 45/06***(2006.01)i; ***A61P 35/00***(2006.01)i; ***A61P 43/00***(2006.01)i; ***A61K 31/439***(2006.01)i; ***A61K 31/4439***(2006.01)i; ***A61K 31/4545***(2006.01)i; ***A61K 31/4709***(2006.01)i; ***A61K 31/496***(2006.01)i; ***A61K 31/506***(2006.01)i; ***A61K 31/517***(2006.01)i; ***A61K 31/519***(2006.01)i; ***A61K 31/5377***(2006.01)i
FI: A61K45/06; A61P35/00; A61P43/00 121; A61K31/4545; A61K31/5377; A61K31/506; A61K31/439; A61K31/517; A61K31/4709; A61K31/519; A61K31/4439; A61K31/496

According to International Patent Classification (IPC) or to both national classification and IPC

**B. FIELDS SEARCHED**

Minimum documentation searched (classification system followed by classification symbols)

A61K45/06; A61P35/00; A61P43/00; A61K31/439; A61K31/4439; A61K31/4545; A61K31/4709; A61K31/496; A61K31/506; A61K31/517; A61K31/519; A61K31/5377

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2020/251965 A1 (BOARD OF REGENTS, THE UNIVERSITY OF TEXAS SYSTEM) 17 December 2020 (2020-12-17)<br>example 6 | 1-21 |
| Y | PLUMMER, Ruth et al. First-in-human study of the PARP/tankyrase inhibitor E7449 in patients with advanced solid tumours and evaluation of a novel drug-response predictor. British Journal of Cancer. 2020, vol. 123, pp. 525-533, https://doi.org/10.1038/s41416-020-0916-5<br>abstract, p. 526, left column, line 5 | 1-21 |
| Y | WANG, Yali et al. PARP inhibitors in gastric cancer: beacon of hope. Journal of Experimental & Clinical Cancer Research. 2021, vol. 40, no. 211, pp. 1-13, https://doi.org/10.1186/s13046-021-02005-6<br>table 2 | 1-21 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 November 2023** | **14 November 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

INTERNATIONAL SEARCH REPORT

International application No. PCT/JP2023/031120

C. DOCUMENTS CONSIDERED TO BE RELEVANT

| Category* | Citation of document, with indication, where appropriate, of the relevant passages | Relevant to claim No. |
|---|---|---|
| Y | THORSELL, Anngerad et al. Structural Basis for Potency and Promiscuity in Poly(ADP-ribose)Polymerase (PARP) and Tankyrase Inhibitors. Journal of Medicinal Chemistry. 2016, vol. 60, pp. 1262-1271, http://dx.doi.org/10.1021/acs.jmedchem.6b00990<br>abstract, table 3 | 1-21 |
| Y | ZAMUDIO-MARTINEZ, Esteban et al. Tankyrases as modulators of pro-tumoral functions: molecular insights and therapeutic opportunities. Journal of Experimental & Clinical Cancer research. 2021, vol. 40, no. 144, pp. 1-15, https://doi.org/10.1186/s13046-021-01950-6<br>table 2, pp. 9-10 | 1-21 |
| Y | JANNE, Psi A. et al. Combined Pan-HER and ALK/ROS1/MET Inhibition with Dacomitinib and Crizotinib in Advanced Non Small Cell Lung Cancer: Results of a Phase I Study. Journal of Thoracic Oncology. 2016, vol. 11 no. 5, pp. 737-747, http://dx.doi.org/10.1016/j.jtho.2016.01.022<br>abstract, p. 739, Biomaker Studies | 2, 7, 10, 16, 18, 20 |

Form PCT/ISA/210 (second sheet) (January 2015)

**INTERNATIONAL SEARCH REPORT**
**Information on patent family members**

International application No.
**PCT/JP2023/031120**

| Patent document cited in search report | Publication date (day/month/year) | Patent family member(s) | Publication date (day/month/year) |
|---|---|---|---|
| WO 2020/251965 A1 | 17 December 2020 | US 2022/0305015 A1<br>example 6 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010143664 A
- WO 2004076412 A
- WO 2006021881 A
- WO 2008073687 A
- WO 2008127248 A
- JP 2008295444 A

### Non-patent literature cited in the description


- *Science*, 04 March 1994, vol. 263 (5151), 1281-4
- *Cancer Res.*, 01 July 2008, vol. 68 (13), 4971-6
- *Cell*, 2010, vol. 141 (1), 69-8
- *Oncogene*, March 2017, vol. 36 (11), 1461-1473
- *Nature Reviews Drug Discovery*, 2012, vol. 11, 923-936
- *Int J Mol Sci.*, 08 July 2021, vol. 22 (14), 7330
- *Sendai Medical Center Journal*, 2019, vol. 9, 23-36
- *Oncogenesis.*, March 2013, vol. 2 (3), e39
- *Precision Oncology*, 2022, vol. 6 (5), 1-12
- *Oncogenesis*, March 2013, vol. 2 (3), e39
- *Expert Opin Ther Pat.*, July 2021, vol. 31 (7), 645-661
- Remington's Pharmaceutical Science. Mack Publishing Company
- *J. Hematol. Oncol.*, 2020, vol. 13, 165
- *Anticancer Drugs.*, 01 February 2022, vol. 33 (2), 124-131
- *Mol Cancer Ther.*, June 2021, vol. 20 (6), 1133-1141